# EUROPÄISCHE PATENTSCHRIFT

(11) **EP 4 561 480 B1**
(45) Veröffentlichungstag und Bekanntmachung des Hinweises auf die Patenterteilung: **24.12.2025**
(21) Anmeldenummer: 24759099.5
(22) Anmeldetag: 20.08.2024
(51) Int. Cl.: A61B 18/14, A61B 17/00, A61B 90/00

(54) **CHIRURGISCHE SCHAFTBAUGRUPPE SOWIE CHIRURGISCHES INSTRUMENT MIT SCHAFTBAUGRUPPE**
SURGICAL SHAFT ASSEMBLY, AND SURGICAL INSTRUMENT COMPRISING SHAFT ASSEMBLY
ENSEMBLE TIGE CHIRURGICALE ET INSTRUMENT CHIRURGICAL COMPRENANT UN ENSEMBLE TIGE

(30) Priorität: 22.08.2023 DE 102023122472
(43) Veröffentlichungstag der Anmeldung: 04.06.2025
(73) Patentinhaber: Aesculap AG, 78532 Tuttlingen (DE)
(72) Erfinder: BARTHELMES, Sven, 78576 Emmingen-Liptingen (DE); ROTHWEILER, Christoph, 78166 Donaueschingen (DE)
(74) Vertreter: Winter, Brandl - Partnerschaft mbB
(86) Internationale Anmeldenummer: PCT/EP2024/073303
(87) Internationale Veröffentlichungsnummer: WO 2025/040660

(56) Entgegenhaltungen:
- EP-A1- 4 018 943
- EP-B1- 3 033 022
- DE-A1- 102010 016 538

## Beschreibung

### Technisches Gebiet

Die vorliegende Offenbarung betrifft eine Schaftbaugruppe für ein chirurgisches Instrument oder eines chirurgischen Instruments, insbesondere elektrochirurgisches Instrument der minimalinvasiven Schaftbauart. Zudem betrifft die vorliegende Offenbarung ein chirurgisches Instrument mit einer solchen Schaftbaugruppe.

Aus dem Stand der Technik sind bereits (elektro-)chirurgische Instrumente, insbesondere der minimalinvasiven Bauart, bekannt, die mittels eines insbesondere mehrteiligen, etwa aus zwei scheren-, klemmbacken-, zangen- oder pinzettenförmigen, gegeneinander/zueinander beweglichen, insbesondere verschwenkbaren Werkzeugbranchen/-elementen aufgebauten Werkzeugs ein Schneiden, Greifen, Halten und/oder Klemmen von Körpergewebe ermöglichen, um dieses durch Anlegen einer Hochfrequenzspannung monopolar oder bipolar zu koagulieren, zu veröden oder zu durchtrennen. Ein solches Instrument ist beispielsweise aus der EP 3 033 022 A1 bekannt.

Auch aus der DE 10 2010 016 538 A1 ist ein Instrument mit einer chirurgischen Schaftbaugruppe, die einen rohrförmigen Instrumentenschaft und einen den Instrumentenschaft ummantelnden, relativ zum Schaft bewegbaren Isolationsmantel aus Kunststoff aufweist.

Dabei ist das Werkzeug an einem distalen (/operateurfernen bzw. patientennahen) Ende des Instruments bzw. der Schaftbaugruppe, insbesondere an einem Instrumentenschaft der Schaftbaugruppe angelenkt (oder anlenkbar) und zu einer Betätigung (/Bedienung) des Werkzeugs über eine Transmission, vorzugsweise in Form einer im Inneren des Instrumentenschafts angeordneten, insbesondere längsverschieblichen Zug-/Druckstange, mit einem Handgriff an einem proximalen (/operateurnahen bzw. patientenfernen) Ende des Instruments bzw. der Schaftbaugruppe gekoppelt. Der Handgriff weist ein der Betätigung des Werkzeugs entsprechendes Betätigungselement (etwa in Form eines Griff-/Abzughebels, einer Taste, eines Drehknopfs, eines Scherengriffs) auf, dessen (insbesondere manuelle) Betätigung/Aktuierung zu einer entsprechend umgesetzten Bewegung der Werkzeugbranchen am Einsatz-/Anwendungsort führt, wie etwa zu einer Schneide-/Greif-/Halte-/Klemmbewegung und/oder einer Dreh-/Schwenkbewegung am oder im Gewebe eines Patienten.

Elektrochirurgische Instrumente der einschlägigen Bauart verwenden in der Regel einen sogenannten Pistolen-Handgriff mit einer starren/unbeweglichen Griffschale, insbesondere in Form eines (Getriebe-)Gehäuse, das sich von distal in Richtung proximal, d.h. im Wesentlichen entlang einer Schaftachse des Instrumentenschafts, erstreckt, und eines feststehenden Griffelements, das sich in einem Winkel/quer zur distal-proximal Richtung erstreckt und an einem proximalen Endabschnitt des (Getriebe-)Gehäuses (integral/unmittelbar) an diesem ausgebildet oder (als separates, fest damit verbundenes Bauteil) daran fixiert ist. An der Griffschale ist ein insbesondere manuell betätigbarer, vorzugsweise fingerführbarer oder fingergeführter Betätigungshebel (/Abzugsbügel/-züngel) schwenkbar angelenkt, der - beispielsweise im Affengriff - durch mehrere Finger einer Greifhand eines Operateurs/Bedieners gehalten und zum Betätigen hin zur Griffschale, insbesondere zum Griffelement, manuell gezogen werden kann. Diese Zieh-/Betätigungsbewegung des Betätigungshebels/Abzugsbügels wird über ein im (Getriebe-)Gehäuse untergebrachtes Getriebe auf die Transmission innerhalb des an den Handgriff angekuppelten oder ankuppelbaren Instrumentenschafts auf das Werkzeug das Instrument übertragen, um dieses entsprechend zu bewegen/betätigen. Außerdem ist an dem Pistolen-Handgriff vorzugsweise eine Art Schalter angebracht, mittels dem das Anlegen der Hochfrequenzspannung an dem Werkzeug ausgelöst werden kann.

Wenigstens eine der beiden Werkzeugbranchen kann mit einer Elektrode oder Elektrodenreihe ausgestattet sein, über welche die Hochfrequenzspannung in das ergriffene Patientengewebe wahlweise eingeleitet werden kann. In diesem Fall läge ein elektrochirurgisches Instrument der monopolaren Bauart vor, bei der ein Patient beispielsweise auf einer Metallplatte aufliegt, über die der Hochfrequenzspannung abgeleitet wird. Alternativ hierzu können aber auch beide, sich gegenüberliegenden Werkzeugbranchen mit einer entsprechenden Elektrode oder Elektrodenreihe ausgestattet sein oder aus einem elektrisch leitfähigen Material bestehen, so dass die Hochfrequenzspannung nur in einem Spalt zwischen den Branchen anliegt. In diesem Fall läge dann ein elektrochirurgisches Instrument der bipolaren Bauart vor.

Über den insbesondere aus einem Metall, vorzugsweise aus Stahl ausgebildeten Instrumentenschaft (oder die im Inneren des Instrumentenschafts angeordnete Zug-/Druckstange) kann ein insbesondere an dem proximalen Ende der Schaftbaugruppe angeordneter Hochfrequenzanschluss elektrisch mit dem Werkzeug verbunden sein oder werden. Um einen elektrischen Kontakt zwischen dem Instrumentenschaft und dem Patienten zu vermeiden, ist der Instrumentenschaft von einem Isolationsmantel (/rohr), vorzugsweise umfangsseitig vollständig, radial außenseitig (elektrisch isolierend) ummantelt (/umschlossen/umgeben). Dazu ist der Isolationsmantel insbesondere aus einem Kunststoff, vorzugsweise aus PEEK ausgebildet.

Um die Schaftbaugruppe bzw. das Instrument (wieder-)verwenden zu können, muss die Schaftbaugruppe, insbesondere ihre mit dem Patienten in Kontakt kommenden Komponenten, wie der Isolationsmantel, sowie die das daran angelenkte Werkzeug vor (je)der Benutzung sterilisiert werden. Die Sterilisation kann insbesondere mittels Erwärmung erfolgen, wodurch sich einzelne Komponenten der Schaftbaugruppe entsprechend ihrem materialabhängigen Wärmeausdehnungsverhalten unterschiedlich ausdehnen können. Die Schaftbaugruppe bzw. das Instrument wird in einem (end)montierten Zustand sterilisiert, um eine anschließende Kontamination durch eine Montage der Schaftbaugruppe zu vermeiden. Deshalb muss zwischen miteinander verbundenen, sich unterschiedlich ausdehnenden Komponenten der Schaftbaugruppe ein die unterschiedliche Ausdehnung zulassendes/ermöglichendes Bewegungsspiel vorhanden sein, um eine Beschädigung der Komponenten zu verhindern. Insbesondere dehnen sich der Instrumentenschaft und der auf dem Instrumentenschaft aufgenommene Isolationsmantel aufgrund ihrer hinsichtlich des Wärmeausdehnungsverhaltens unterschiedlichen Materialien unterschiedlich weit aus, weshalb der Isolationsmantel axial verschieblich auf dem Instrumentenschaft angeordnet ist. Jedoch kann es aufgrund der axial beweglichen Anordnung (und dadurch, dass sich der Isolationsmantel beim Abkühlen nach der Sterilisation nicht zwangsweise in seine ursprüngliche Position vor dem Erwärmen zurückzieht) zu einem (axialen) Verrutschen des Isolationsmantels relativ zu dem Instrumentenschaft kommen. Insbesondere kann dabei an dem distalen Ende der Schaftbaugruppe ein Axialspalt zwischen dem Instrumentenschaft und dem Isolationsmantel entstehen, was unbedingt zu vermeiden ist.

Der vorliegenden Offenbarung liegt daher die Aufgabe zu Grunde, eine Schaftbaugruppe eines oder für ein chirurgisches Instrument sowie ein chirurgisches Instrument mit einer solchen Schaftbaugruppe, die sterilisierbar ist und den hohen Sicherheitsanforderungen genügt.

Die Aufgabe wird durch eine chirurgische Schaftbaugruppe eines oder für ein chirurgisches Instrument, insbesondere elektrochirurgisches Instrument mit den Merkmalen des unabhängigen Patentanspruchs und/oder durch ein chirurgisches Instrument, insbesondere elektrochirurgisches Instrument mit den Merkmalen des nebengeordneten Patentanspruchs gelöst. Vorteilhafte Weiterbildungen sind Gegenstand der Unteransprüche.

Insbesondere wird die Aufgabe durch eine Schaftbaugruppe für ein chirurgisches, insbesondere elektrochirurgisches Instrument oder eines chirurgischen, insbesondere elektrochirurgischen Instruments gelöst.

An einem distalen Ende (/Arbeitsende) der Schaftbaugruppe bzw. des Instruments ist ein Werkzeug anlenkbar oder angelenkt und an einem proximalen Ende (/Betätigungsende) der Schaftbaugruppe bzw. des Instruments ist ein Handgriff zur Betätigung des Werkzeugs, insbesondere über die Schaftbaugruppe, anlenkbar oder angelenkt.

Die Schaftbaugruppe weist einen insbesondere aus einem Metall, vorzugsweise aus Stahl, ausgebildeten Instrumentenschaft auf, sowie einen aus einem (hinsichtlich eines Wärmeausdehnungsverhaltens) unterschiedlichen Material als der Instrumentenschaft, insbesondere aus Kunststoff, vorzugsweise aus PEEK, ausgebildeten Isolationsmantel (/-rohr), der axial verschieblich auf dem Instrumentenschaft angeordnet ist und den Instrumentenschaft radial außenseitig zur elektrischen Isolierung des Instrumentenschafts, vorzugsweise umfangsseitig vollständig, ummantelt (/umschließt/umgibt).

Gemäß einem Aspekt der vorliegenden Offenbarung weist die Schaftbaugruppe einen axial verschieblich auf dem Instrumentenschaft aufgenommenen (/angeordneten) und in eine distale Richtung axialvorgespannten, proximalen Axialanschlag zur Begrenzung einer proximal gerichteten Axialbewegung des Isolationsmantels relativ zu dem Instrumentenschaft auf. Das heißt, dass der Axialanschlag, an dem der Isolationsmantel auf seiner proximalen Seite anliegt und damit in seiner Axialverlagerung (bzw. Axialausdehnung) (durch eine Axialposition des proximalen Axialanschlags) in eine proximale Richtung begrenzt ist, (ebenso wie der Isolationsmantel) relativ zu dem Instrumentenschaft axial beweglich ist. Dabei wirkt eine distal gerichtete Vorspannkraft/Axialkraft auf den Axialanschlag (und damit auf den Isolationsmantel), so dass der Axialanschlag (und damit auch der Isolationsmantel) in die distale Richtung (/in Richtung zum Werkzeug hin) gedrückt wird. Dadurch kann ein sich durch das Verrutschen des Isolationsmantels gebildeter Axialspalt geschlossen werden.

Mit anderen Worten liegt der Kern der Offenbarung darin, dass sich der proximale Axialanschlag, insbesondere bei bzw. aufgrund einer wärmebedingter Ausdehnung des Isolationsmantels, axial auf dem Instrumentenschaft entgegen der Vorspannung in die proximale Richtung bewegen kann, um (dann), insbesondere bei einem abkühlbedingten Schrumpfen/Zusammenziehen und dadurch entstehenden Verrutschen/unkontrollierten Verlagern des Isolationsmantels, durch die Vorspannung in die distale Richtung zurückbewegt zu werden und dadurch den Isolationsmantel in seine ursprüngliche Montageposition zu verschieben.

Gemäß der vorliegenden Offenbarung kann die Schaftbaugruppe zwei der (axial verschieblich auf dem Instrumentenschaft aufgenommenen und in die distale Richtung axialvorgespannten) proximalen Axialanschläge aufweisen, von denen ein erster proximaler Axialanschlag zur Begrenzung der Axialbewegung des Isolationsmantels mit einem ersten (kleineren, beispielsweise 5 Millimeter großen) Durchmesser und ein zweiter proximaler Axialanschlag zur Begrenzung der Axialbewegung des Isolationsmantels mit einem zweiten (größeren, beispielsweise 10 Millimeter großen) Durchmesser dient. Dies hat den Vorteil, dass die(selbe) Schaftbaugruppe sowohl mit einem Isolationsmantel mit einem kleineren Durchmesser als auch mit einem Isolationsmantel mit einem größeren Durchmesser kompatibel ist. Das heißt, dass die Schaftbaugruppe universell und modular einsetzbar ist. Insbesondere kann die Schaftbaugruppe dabei immer nur mit einem Isolationsmantel eingesetzt werden.

Gemäß einer bevorzugten Ausführungsform kann die Schaftbaugruppe einen distalen Axialanschlag zur Begrenzung einer distal gerichteten Axialbewegung des Isolationsmantels relativ zu dem Instrumentenschaft aufweisen. Vorzugsweise kann der distale Axialanschlag axialfest mit dem Instrumentenschaft verbunden sein. Dies hat den Vorteil, dass der Isolationsmantel einen definierten Endanschlag für die Verschiebung in die distale Richtung hat und somit nicht durch den axialvorgespannten proximalen Axialanschlag zu weit, d.h. über seine ursprüngliche Montageposition hinaus, in die distale Richtung verlagert werden kann.

Gemäß einer bevorzugten Ausführungsform kann die Schaftbaugruppe eine insbesondere auf dem Instrumentenschaft aufgenommene, vorzugsweise ringförmige, den proximalen Axialanschlag ausbildende Scheibe aufweisen. Beispielsweise kann ein Innendurchmesser der (Ring-)Scheibe (geringfügig) größer als ein Außendurchmesser des Instrumentenschaft sein. So kann eine geführte, aber axial lose Aufnahme des proximalen Axialanschlags konstruktiv besonders einfach realisiert werden.

Gemäß einer bevorzugten Ausführungsform kann die Schaftbaugruppe ein den proximalen Axialanschlag axial vorspannendes Druckelement, insbesondere in Form einer Feder, vorzugsweise Schraubenfeder, aufweisen. Das Druckelement kann insbesondere axial zwischen dem proximalen Axialanschlag und einem axialfest mit dem Instrumentenschaft verbundenen oder direkt/unmittelbar an dem Instrumentenschaft ausgebildeten Federanschlag angeordnet sein. Dadurch kann die axiale Vorspannung auf den proximalen Axialanschlag aufgebracht werden.

Gemäß einer Weiterbildung der bevorzugten Ausführungsform kann das Druckelement an der Scheibe oder direkt an dem Isolationsmantel anliegen. So kann das Druckelement die axiale Vorspannung auf die als der Axialanschlag fungierende Scheibe und damit mittelbar auf den Isolationsmantel aufbringen, was Vorteile hinsichtlich einer gleichmäßigen Krafteinleitung hat, oder alternativ die axiale Vorspannung direkt auf den Isolationsmantel aufbringen und damit selbst (bzw. seine Axialstirnfläche) als der Axialanschlag fungieren, was Vorteile hinsichtlich einer Reduktion einer Bauteilanzahl hat.

Gemäß einer bevorzugten Ausführungsform kann die Schaftbaugruppe einen vorzugsweise axialfest mit dem Instrumentenschaft verbundenen, insbesondere auf dem Instrumentenschaft aufgenommenen, vorzugsweise den Instrumentenschaft außenseitig elektrisch isolierenden, kapselartigen Adapter aufweisen, (radial) innerhalb welchem der proximale Axialanschlag, d.h. insbesondere die Scheibe und/oder das Druckelement, angeordnet ist. Dies hat den Vorteil, dass der Axialanschlag und damit auch das proximale Ende des Isolationsmantels eingehaust sind und somit keinen Kontakt zur Umgebung haben.

Gemäß einer Weiterbildung der bevorzugten Ausführungsform kann der Adapter einen distalen (Scheiben-)Anschlag, insbesondere in Form eines sich radial nach innen erstreckenden Abschnitts, zur Begrenzung einer distal gerichteten Axialbewegung des (axial verschieblichen) proximalen Axialanschlags, d.h. insbesondere der Scheibe und/oder des Druckelements, aufweisen. Dies hat den Vorteil, dass der proximale Axialanschlag einen definierten Endanschlag für die Verschiebung in die distale Richtung hat und somit nicht durch den axialvorgespannten proximalen Axialanschlag zu weit in die distale Richtung verlagert werden kann, sondern an einer vorbestimmten Position positioniert wird.

Gemäß der Weiterbildung der bevorzugten Ausführungsform kann eine Axialposition des distalen (Scheiben-)Anschlags in Abhängigkeit eines Wärmeausdehnungsverhaltens des Isolationsmantels und/oder des Instrumentenschafts festgelegt sein. Insbesondere kann die Axialposition dabei so festgelegt sein, dass das proximale Ende des Isolationsmantels im abgekühlten Zustand innerhalb des Adapters liegt bzw. dass der Isolationsmantel im abgekühlten Zustand sowohl am (insbesondere Instrumentenschaftfesten) distalen Axialanschlag als auch am proximalen Axialanschlag anliegt (und nicht etwa ein Axialspalt zwischen dem Isolationsmantel und dem distalen oder proximalen Axialanschlag dadurch entsteht, dass der proximale Anschlag aufgrund seines distalen (Scheiben-)Anschlags nicht ausreichend weit in die distale Richtung geschoben werden kann.

Gemäß einer Weiterbildung der bevorzugten Ausführungsform kann der Adapter eine Aufnahmeschale mit einer Einlegeöffnung zum Einlegen des proximalen Axialanschlags, d.h. insbesondere der Scheibe und/oder des Druckelements, aufweisen. Dabei kann die Einlegeöffnung auf einer proximalen Seite oder einer distalen Seite des Adapters ausgebildet sein. Vorzugsweise kann die Einlegeöffnung einen Innendurchmesser aufweisen, der größer als ein Außendurchmesser des proximalen Axialanschlags, d.h. insbesondere der Scheibe und/oder des Druckelements, ist. Dadurch wird gewährleistet, dass der proximale Axialanschlag montierbar ist.

Gemäß der Weiterbildung der bevorzugten Ausführungsform kann der Adapter eine insbesondere separat von der Aufnahmeschale ausgebildete, von einer distalen Seite auf die Aufnahmeschale aufsteckbare (oder aufgesteckte), insbesondere aufschraubbare (oder aufgeschraubte), den distalen Anschlag ausbildende Kappe aufweisen. Durch die mehrteilige Ausbildung ergeben sich konstruktive Vorteile hinsichtlich Funktionalität und Montierbarkeit.

Gemäß der Weiterbildung der bevorzugten Ausführungsform kann der distale Anschlag alternativ direkt/integral an einer distalen Seite der Aufnahmeschale ausgebildet sein. Das heißt, dass der proximale Axialanschlag, d.h. insbesondere die Scheibe und/oder das Druckelement, von der proximalen Seite in die Aufnahmeschale (durch die proximal angeordnete Einlegeöffnung) eingeschoben werden und (direkt/unmittelbar) an einer Wandung der Aufnahmeschale anliegen. Dies hat den Vorteil, dass eine einfache Ausgestaltung des Adapters möglich ist.

Gemäß der Weiterbildung der bevorzugten Ausführungsform kann der Adapter eine distale Öffnung/Durchführöffnung aufweisen, deren Außendurchmesser im Wesentlichen einem Außendurchmesser des Isolationsmantels entspricht und durch die der Isolationsmantel und der Instrumentenschaft hindurchführbar sind. Alternativ kann der Adapter eine zentrale Öffnung/Durchführöffnung, deren Außendurchmesser im Wesentlichen einem Außendurchmesser des Instrumentenschafts entspricht und durch die der Instrumentenschaft axial hindurchführbar ist, sowie eine zweite Durchführöffnung aufweisen, durch die der Isolationsmantel (zur Kontaktierung des proximalen Axialanschlags) axial hindurchführbar ist. Dadurch kann die Funktionalität des innerhalb des Adapters angeordneten proximalen Axialanschlags gewährleistet werden.

Gemäß der Weiterbildung der bevorzugten Ausführungsform kann die distale Öffnung alternativ (direkt/integral) an einem Innendurchmesser/Innenumfang der Aufnahmeschale ausgebildet sein. Dies hat den Vorteil, dass die distale Seite der Aufnahmeschale im Wesentlichen (mit Ausnahme der distale/zentrale Öffnung) integral geschlossen (/verschlossen) ausgebildet sein kann.

Gemäß der Weiterbildung der bevorzugten Ausführungsform kann der Adapter einen die Einlegeöffnung verschließenden und an seinem Innenumfang die distale/zentrale Öffnung ausbildenden Axialdeckel aufweisen. Dies hat den Vorteil, dass die distale Seite der Aufnahmeschale im Wesentlichen (mit Ausnahme der distale/zentrale Öffnung) geschlossen ist, so dass das Innere keinen Kontakt zur Umgebung hat.

Gemäß der Weiterbildung der bevorzugten Ausführungsform kann der Axialdeckel durch dem proximalen Axialanschlag, d.h. insbesondere Scheibe, gebildet sein. So lässt sich eine Funktionsintegration und damit eine Reduzierung der Bauteilanzahl erreichen.

Gemäß einer Weiterbildung der bevorzugten Ausführungsform kann die Schaftbaugruppe ein den ersten proximalen Axialanschlag axial vorspannendes erstes Druckelement, insbesondere in Form einer Feder, vorzugsweise Schraubenfeder, und ein den zweiten proximalen Axialanschlag axial vorspannendes zweites Druckelement, insbesondere in Form einer Feder, vorzugsweise Schraubenfeder, aufweisen.

Gemäß der Weiterbildung der bevorzugten Ausführungsform können das erste Druckelement und das zweite Druckelement unterschiedliche Federhärten aufweisen. So kann eine an den jeweiligen axial durch die Vorspannung zu verschiebenden Isolationsmantel angepasste Vorspannkraft realisiert werden. Vorzugsweise kann die Federhärte des zweiten Druckelements (für den größeren Isolationsmantel) größer als die Federhärte des ersten Druckelements (für den kleineren Isolationsmantel) sein.

Gemäß der Weiterbildung der bevorzugten Ausführungsform können das erste Druckelement und das zweite Druckelement radial geschachtelt angeordnet sein. So lässt sich eine bauraumsparende Anordnung realisieren. Vorzugsweise kann das erste Druckelement radial innerhalb des zweiten Druckelements angeordnet sein. Dies hat den Vorteil, dass aufgrund der größeren Federhärte oftmals ein größeres Druckelement erforderlich ist, so dass das erste Druckelement einfacher innerhalb des zweiten Druckelements angeordnet werden kann als umgekehrt.

Gemäß einer Weiterbildung der bevorzugten Ausführungsform kann die Schaftbaugruppe eine den ersten proximalen Axialanschlag ausbildende, vorzugsweise ringförmige, erste Scheibe und eine den zweiten proximalen Axialanschlag ausbildende, vorzugsweise ringförmige, zweite Scheibe aufweisen.

Gemäß der Weiterbildung der bevorzugten Ausführungsform können die erste Scheibe und die zweite Scheibe axial hintereinander, insbesondere aneinander anliegend, angeordnet sein. Vorzugsweise kann die zweite Scheibe proximal der ersten Scheibe angeordnet sein. So lässt sich eine bauraumsparende Anordnung realisieren.

Gemäß der Weiterbildung der bevorzugten Ausführungsform kann der Axialdeckel durch die proximal angeordnete Scheibe, insbesondere die zweite Scheibe, gebildet sein. So lässt sich die Funktion des Axialdeckels in die proximal angeordnete Scheibe integrieren.

Gemäß der Weiterbildung der bevorzugten Ausführungsform kann die distal angeordnete Scheibe einen solchen Innendurchmesser oder eine solche Durchführöffnung (in ihrem Ringabschnitt) aufweisen, durch den oder die der Isolationsmantel mit entsprechendem Durchmesser zum axialen Anliegen an der proximal angeordneten Scheibe (/zum Kontaktieren des entsprechenden proximalen Axialanschlags) axial hindurchführbar ist. So wird trotz der Hintereinander-Anordnung der Scheiben gewährleistet, dass jede Scheibe von dem Isolationsmantel mit entsprechendem Durchmesser kontaktiert werden kann.

Gemäß einer bevorzugten Ausführungsform kann die Schaftbaugruppe einen dritten proximalen Axialanschlag zur Begrenzung der Axialbewegung des Isolationsmantels mit einem dritten (mittleren, beispielsweise 8 Millimeter großen) Durchmesser aufweisen. Gemäß einer Weiterbildung kann ein den dritten proximalen Axialanschlag axial vorspannendes drittes Druckelement, insbesondere in Form einer Feder, vorzugsweise Schraubenfeder, eine unterschiedliche Federhärte als das erste und/oder zweite Druckelement aufweisen. Insbesondere kann das dritte Druckelement eine größere Federhärte als das erste Druckelement und/oder eine kleinere Federhärte als das dritte Druckelement aufweisen. Gemäß einer Weiterbildung kann das dritte Druckelement radial geschachtelt mit dem ersten und/oder zweiten Druckelement angeordnet sein. Insbesondere kann das dritte Druckelement radial außerhalb des ersten Druckelements und/oder des zweiten Druckelements (oder radial zwischen dem ersten Druckelement und dem zweiten Druckelement) angeordnet sein. Gemäß einer Weiterbildung kann eine den dritten proximalen Axialanschlag ausbildende dritte Scheibe proximal der ersten und/oder zweiten Scheibe, axial zwischen der ersten und zweiten Scheibe, oder distal der ersten und/oder zweiten Scheibe angeordnet sein. Insbesondere kann die dritte Scheibe axial zwischen der ersten Scheibe und der zweiten Scheibe angeordnet sein.

Die Aufgabe der Offenbarung wird auch durch ein chirurgisches Instrument, insbesondere elektrochirurgisches Instrument der minimalinvasiven Schaftbauart, mit einer beschriebenen Schaftbaugruppe gelöst.

### Kurzbeschreibung der Figuren

Fig. 1 zeigt eine perspektivische Ansicht eines Instruments gemäß der vorliegenden Offenbarung,
Fig. 2 zeigt eine perspektivische Ansicht eines distalen Teils des Instruments,
Fig. 3 zeigt eine vergrößerte perspektivische Ansicht eines proximalen Teils des Instruments,
Fign. 4 bis 11 zeigen eine Ausbildung bzw. Aufnahme eines Isolationsmantels des Instruments;
Fig. 12 zeigt eine Längsschnittdarstellung eines Handgriffs des Instruments;
Fign. 13 und 14 zeigen Längsschnittdarstellungen des Handgriffs in einer vollständig geöffneten bzw. geschlossenen Stellung (bzw. betätigten und unbetätigten Stellung) eines Werkzeugs des Instruments;
Fign. 15 und 16 zeigen eine Überlastsicherungsfunktion des Instruments;
Fig. 17 zeigt eine Längsschnittdarstellung des Handgriffs des Instruments in einer Ladeposition;
Fig. 18 zeigt eine Längsschnittdarstellung eines distalen Endbereichs des Instruments; und
Fig. 19 zeigt eine Querschnittsdarstellung im Bereich eines Demontageknopfs des Instruments.

### Beschreibung von bevorzugten Ausführungsformen

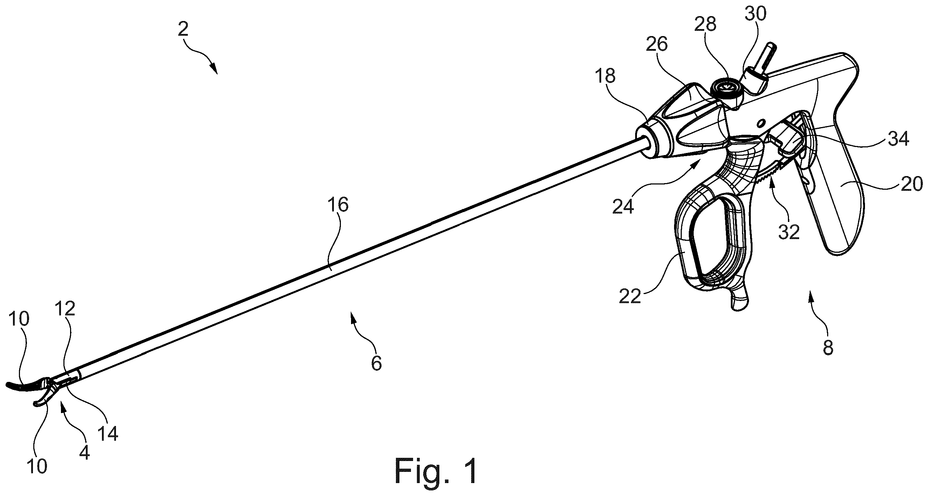

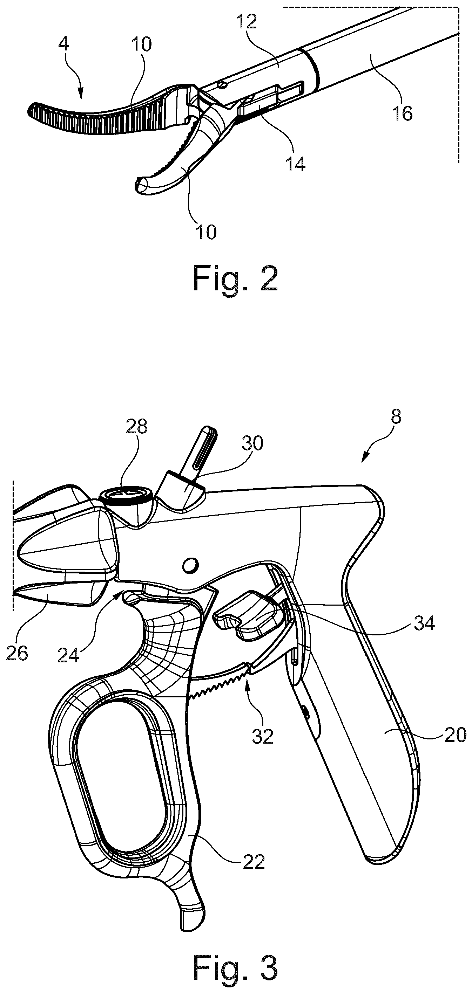

Fig. 1 zeigt eine perspektivische Ansicht eines chirurgischen Instruments 2 gemäß der vorliegenden Offenbarung. Fign. 2 und 3 zeigen vergrößerte perspektivische Ansichten eines distalen Teils bzw. proximalen Teils des Instruments 2. Das Instrument 2 ist insbesondere als ein elektrochirurgisches Instrument ausgebildet und zum Einsatz in der minimalinvasiven Chirurgie bzw. Endoskopie, insbesondere Laparoskopie vorgesehen. Das Instrument 2 ist insbesondere als ein Instrument 2 der minimalinvasiven Schaftbauart ausgebildet.

Das Instrument 2 weist ein distal angeordnetes Werkzeug 4, eine proximal zu dem Werkzeug 4 angeordnete Schaftbaugruppe 6 und einen proximal (zu dem Werkzeug 4 und der Schaftbaugruppe 6) angeordneten Handgriff 8 auf. Das heißt, dass das Werkzeug 4 an einem distalen Ende (/Arbeitsende) der Schaftbaugruppe 6 ankoppelbar oder angekoppelt ist und ein proximales Ende (/Betätigungsende) der Schaftbaugruppe 6 an dem Handgriff 8 distal ankoppelbar/ankuppelbar oder angekoppelt/angekuppelt ist. Dabei sind proximal und distal auf einen Operateur (/Bediener/Betätiger/Benutzer) des Instruments 2 bezogen definiert.

Das Instrument 2 weist das (distal angeordnete) Werkzeug 4 auf. Das Werkzeug 4 ist insbesondere mehrteilig aufgebaut und kann beispielsweise aus zwei schere-, klemmbacken-, zangen- oder pinzettenförmigen, gegeneinander/zueinander beweglichen, insbesondere zueinander verschwenkbaren Werkzeugbranchen (/elementen) 10 aufgebaut sein. Bei einem Betätigen des Werkzeugs 4 verschwenken die Werkzeugbranchen 10 relativ zueinander, wodurch sie sich öffnen bzw. schließen. Das Werkzeug 4 kann bzw. die Werkzeugbranchen 10 können zum Schneiden, Greifen, Halten und/oder Klemmen von Körpergewebe dienen. Die Werkzeugbranchen 10 sind insbesondere an der Schaftbaugruppe 6 um eine Werkzeugschwenkachse drehbar angelenkt, so dass zumindest eine der Werkzeugbranchen 10, vorzugsweise beide Werkzeugbranchen 10, relativ zu der Schaftbaugruppe 6 und damit auch relativ zu der jeweils anderen Werkzeugbranche 10 verschwenkt werden kann. Die Werkzeugschwenkachse ist insbesondere quer bzw. senkrecht zu einer distal-proximal-Richtung ausgerichtet. Die distal-proximal-Richtung entspricht insbesondere einer Längsachse der Schaftbaugruppe 6 (im Folgenden lediglich als eine Schaftachse bezeichnet). Das Werkzeug 4 ist insbesondere um seine Längsachse drehgekoppelt mit der Schaftbaugruppe 6 verbunden, so dass das Werkzeug 4 (als Ganzes) mit der Schaftbaugruppe 6 verdreht werden kann. Das Werkzeug 4 ist insbesondere aus einem Metall, vorzugsweise aus Stahl, ausgebildet.

Das Instrument 2 bzw. die Schaftbaugruppe 6 weist einen Instrumentenschaft (/Rohrschaft) 12 auf, dessen Längsachse (/Rohrachse) insbesondere der Schaftachse entspricht. Der Instrumentenschaft 12 kann vorzugsweise translatorisch fest und vorzugsweise um die Schaftachse drehbar aufgenommen sein. Das Werkzeug 4 kann mit dem Instrumentenschaft 12 derart gekoppelt sein, dass eine Rotation des Instrumentenschafts 12 (um die Schaftachse) eine Rotation des Werkzeugs 4 (um die Schaftachse) hervorruft (/erzwingt/aktuiert). Insbesondere können das Werkzeug 4 und der Instrumentenschaft 12, vorzugsweise direkt, um die Schaftachse drehfest miteinander verbunden sein. Der Instrumentenschaft 12 ist insbesondere aus einem Metall, vorzugsweise aus Stahl, ausgebildet.

Das Instrument 2 bzw. die Schaftbaugruppe 6 weist eine Transmission, vorzugsweise eine im Inneren/innerhalb des Instrumentenschafts 12 gelagerte (/aufgenommene/angeordnete) Zug-/Druckstange 14 auf, deren Längsachse (/Stangenachse) insbesondere der Schaftachse bzw. im Wesentlichen der distal-proximal-Richtung entspricht. Die Transmission (/die Zug-/Druckstange 14) kann translatorisch verschiebbar, vorzugsweise axial-/längsbeweglich/-verschieblich, d.h. translatorisch entlang der Schaftachse verschieblich, und vorzugsweise rotatorisch fest aufgenommen sein. Das Werkzeug 4 kann mit der Transmission (/Zug-/Druckstange 14) derart gekoppelt sein, dass eine Translationsbewegung, insbesondere Längsbewegung (entlang der Schaftachse) ein Betätigen des Werkzeugs 4, insbesondere eine Schwenkbewegung (bzw. Öffnen und Schließen) der Werkzeugbranchen 10 (um die Werkzeugschwenkachse), hervorruft (/erzwingt/aktuiert). Insbesondere können das Werkzeug 4 und die Zug-/Druckstange 14, vorzugsweise über einen Kopplungsmechanismus, miteinander verbunden sein. Das heißt, dass die Zug-/Druckstangen-Längsbewegung in eine distale Richtung/Schubrichtung das Öffnen (oder Schließen) des Werkzeugs 4 bzw. der Werkzeugbranchen 10 und in eine proximale Richtung/Zugrichtung das Schließen (oder Öffnen) des Werkzeugs 4 bzw. der Werkzeugbranchen 10 hervorruft (/erzwingt/aktuiert). Die Zug-/Druckstange 14 ist insbesondere aus einem Metall, vorzugsweise aus Stahl, ausgebildet.

Das Instrument 2 bzw. die Schaftbaugruppe 6 weist einen insbesondere auf dem Instrumentenschaft 12 angeordneten Isolationsmantel 16 auf, dessen Längsachse (/Mantelachse) insbesondere der Schaftachse entspricht. Der Isolationsmantel 16 kann, insbesondere relativ zu dem Instrumentenschaft 12 und/oder der Zug-/Druckstange 14, axial-/längsbeweglich/-verschieblich, d.h. translatorisch entlang der Schaftachse verschieblich, und vorzugsweise (frei) drehbar aufgenommen sein. Der Isolationsmantel 16 ist hohl, vorzugsweise rohrförmig, ausgebildet und dient zur (radial) außenseitigen elektrischen Isolierung des Instrumentenschafts 12 und/oder der Zug-/Druckstange 14, insbesondere zwischen einem distalen Endbereich und einem proximalen Endbereich des Instrumentenschafts 12 und/oder der Zug-/Druckstange 14. Der Isolationsmantel 16 ist aus einem zum Instrumentenschaft 12 unterschiedliche Material, insbesondere aus einem Kunststoff, vorzugsweise aus PEEK, ausgebildet. Eine Ausbildung bzw. Aufnahme des Isolationsmantels 16 wird untenstehend näher beschrieben.

Das Instrument 2 bzw. die Schaftbaugruppe 6 weist eine insbesondere auf dem Instrumentenschaft 12 angeordnete, vorzugsweise im Querschnitt ringförmige Kappe 18 auf. Durch eine (zentrale) Öffnung der Kappe 18 können der Instrumentenschaft 12, die Zug-/Druckstange 14 und/oder der Isolationsmantel 16 axial hindurchgeführt sein. Die Kappe 18 kann, insbesondere relativ zu dem Instrumentenschaft 12, translatorisch fest und vorzugsweise rotatorisch fest aufgenommen sein. Die Kappe 18 dient als Axialanschlag der Schaftbaugruppe 6 an dem Handgriff 8.

Das Instrument 2 weist den (proximal angeordneten) Handgriff 8 auf. Der Handgriff 8 ist insbesondere nach Art eines Pistolengriffs bzw. als ein Pistolen-Handgriff ausgebildet. Der Handgriff 8 weist ein Getriebegehäuse 20 auf, das sich insbesondere von distal in Richtung proximal, d.h. in der distal-proximal-Richtung bzw. im Wesentlichen parallel zur/entlang der Schaftachse, erstreckt.

Zudem weist der Handgriff 8 ein feststehendes Griffelement (/Griffteil) 21 auf. Das Griffelement 21 erstreckt sich insbesondere in einem Winkel, d.h. quer, zur distal-proximal-Richtung. Das Griffelement 21 kann an einem proximalen Endabschnitt des Getriebegehäuses 20 fixiert sein oder insbesondere an dem Getriebegehäuse 20 ausgebildet sein, d.h. integral mit dem Getriebegehäuse 20 verbunden sein. Insbesondere sind das Getriebegehäuse 20 und das Griffelement 21 fest miteinander verbunden.

Weiter weist der Handgriff 8 einen an dem Getriebegehäuse 20 angelenkten, insbesondere fingergeführten bzw. fingerführbaren Betätigungshebel 22 auf. Der Betätigungshebel 22 ist insbesondere manuell betätigbar und weist eine Angriffsstelle zum Aufbringen einer Betätigungskraft (durch den Operateur) aus. Der Betätigungshebel 22 kann eine, vorzugsweise geschlossene bzw. im Wesentlichen ringförmige, Schlaufe zur Aufnahme von Fingern (vorzugsweise nicht eines Daumens) des Operateurs aufweisen, welche die Betätigungskraft-Angriffsstelle bildet. Der Betätigungshebel 22 kann schwenkbar aufgenommen sein. Der Betätigungshebel 22 ist insbesondere an dem Getriebegehäuse 20 um eine Betätigungshebelschwenkachse drehbar angelenkt, so dass der Betätigungshebel 22 relativ zu dem Getriebegehäuse 20, d.h. zu dem Griffelement 21 hin oder von dem Griffelement 21 weg, verschwenkt werden kann. Die Betätigungshebelschwenkachse ist insbesondere quer bzw. senkrecht zu der Schaftachse, d.h. der distal-proximal-Richtung. Durch eine manuelle Betätigung des Betätigungshebels 22, d.h. durch ein Aufbringen der Betätigungskraft an der Betätigungskraft-Angriffsstelle, insbesondere der Schlaufe, wird eine Schwenkbewegung des Betätigungshebels 22 (relativ zu dem Getriebegehäuse 20) hervorgerufen (/erzwungen/aktuiert). Die Schwenkbewegung des Betätigungshebels 22 zu dem Griffelement 21 hin, aktuiert etwa durch Schließen einer Hand des Operateurs/Zusammendrücken von Betätigungshebel 22 und Griffelement 21, wird im Folgenden lediglich als Verschwenken/Schwenkbewegung in eine Betätigungsrichtung bzw. Betätigen des Betätigungshebels 22 bezeichnet. Die Schwenkbewegung des Betätigungshebels 22 von dem Griffelement 21 weg, aktuiert etwa durch Öffnen einer Hand des Operateurs/Auseinanderdrücken von Betätigungshebel 22 und Griffelement 21, wird im Folgenden lediglich als Verschwenken/Schwenkbewegung in eine Rückstellrichtung bzw. Rückstellen des Betätigungshebels 22 bezeichnet.

Das Instrument 2 bzw. der Handgriff 8 weist ein Getriebe 24 auf, das die (durch die manuelle Betätigung aktuierte) Schwenkbewegung des Betätigungshebels 22 in eine Translationsbewegung der Transmission, insbesondere in eine Längsbewegung der Zug-/Druckstange 14 überträgt. Das heißt, dass das Getriebe 24 die Schwenkbewegung des Betätigungshebels 22 mit der Längsbewegung der Zug-/Druckstange 14 (und damit (indirekt) mit dem Betätigen des Werkzeugs 4 bzw. dem Öffnen und dem Schließen der Werkzeugbranchen 10) koppelt. Mit anderen Worten aktuiert das Betätigen des Betätigungshebels 22 die Längsbewegung der Zug-/Druckstange 14 in die Schubrichtung (oder in die Zugrichtung) und das Rückstellen des Betätigungshebels 22 die Längsbewegung der Zug-/Druckstange 14 in die Zugrichtung (oder in die Schubrichtung) (welche wiederum das Betätigen (bzw. Öffnen oder Schließen) des Werkzeugs 4 hervorruft). Das Getriebe 24 kann vorzugsweise größtenteils oder vollständig innerhalb des Getriebegehäuses 20 angeordnet sein bzw. von dem Getriebegehäuse 20 nach außen hin abgedeckt sein. Eine Ausbildung des Getriebes 24 wird untenstehend näher beschrieben.

Das Instrument 2 bzw. der Handgriff 8 weist einen, insbesondere an einem distalen Ende des Handgriffs 8 angeordneten Drehstern 26 auf, dessen Längsachse (/Sternachse) insbesondere der Schaftachse entspricht. Der Drehstern 26 kann, insbesondere relativ zu dem Getriebegehäuse 20, vorzugsweise translatorisch fest und vorzugsweise um die Schaftachse drehbar aufgenommen sein. Durch eine (zentrale) Öffnung des Drehsterns 26 können der Instrumentenschaft 12, die Zug-/Druckstange 14 und/oder der Isolationsmantel 16 axial hindurchgeführt oder hindurchführbar sein. Der Drehstern 26 kann mit dem Instrumentenschaft 12 derart koppelbar oder gekoppelt sein, dass eine Rotation des Drehsterns 26 (um die Schaftachse) die Rotation des Instrumentenschafts 12 (um die Schaftachse) hervorruft (/erzwingt/aktuiert) (welche wiederum die Rotation des Werkzeugs 4 hervorruft). Insbesondere können der Drehstern 26 und der Instrumentenschaft 12, vorzugsweise direkt oder über ein mit dem Instrumentenschaft 12 (fest) verbundenes Bauteil, um die Schaftachse drehfest miteinander verbunden sein.

Das Instrument 2 bzw. der Handgriff 8 weist einen Demontageknopf 28 auf, bei dessen Betätigung (/Drücken) die Schaftbaugruppe 6 und der Handgriff 8 demontierbar sind, d.h. die Schaftbaugruppe 6 aus dem Handgriff 8 auskoppelbar ist. Eine Ausbildung des Demontageknopfs 28 wird untenstehend näher beschrieben.

Das Instrument 2 bzw. der Handgriff 8 weist einen Hochfrequenzanschluss, insbesondere einen HF-Pin 30 auf, durch den das Werkzeug 4, insbesondere die Werkzeugbranchen 10, mit einer Hochfrequenzspannung beaufschlagbar ist. Der **HF-**Pin 30 kann, insbesondere relativ zu dem Getriebegehäuse 20, vorzugsweise translatorisch fest und vorzugsweise rotatorisch fest aufgenommen sein. Der HF-Pin 30 kann in Kontakt mit dem Instrumentenschaft 12 und/oder der Zug-/Druckstange 14 sein oder bringbar sein, um die Hochfrequenzspannung durch das Material des Instrumentenschafts 12 und/oder der Zug-/Druckstange 14 an das Werkzeug 4 zu übertragen. Der HF-Pin 30 kann als ein bipolarer oder als ein monopolarer HF-Pin ausgebildet sein.

Der Handgriff 8 weist einen Rastmechanismus 32 auf, durch den eine Schwenkposition des Betätigungshebels 22 in vorbestimmten Rastpositionen feststellbar ist. Der Rastmechanismus 32 kann aus einem am Griffelement 21 (fest) angebrachten Rastbügel und einer am Betätigungshebel 22 (fest) angebrachten Raste aufgebaut sein.

Der Handgriff 8 weist einen Taster 34 auf, mit dem eine Beaufschlagung des Werkzeugs 4 mit der Hochfrequenzspannung ausgelöst werden kann. Alternativ kann der Taster 34 zur Entriegelung des Rastmechanismus 32 dienen.

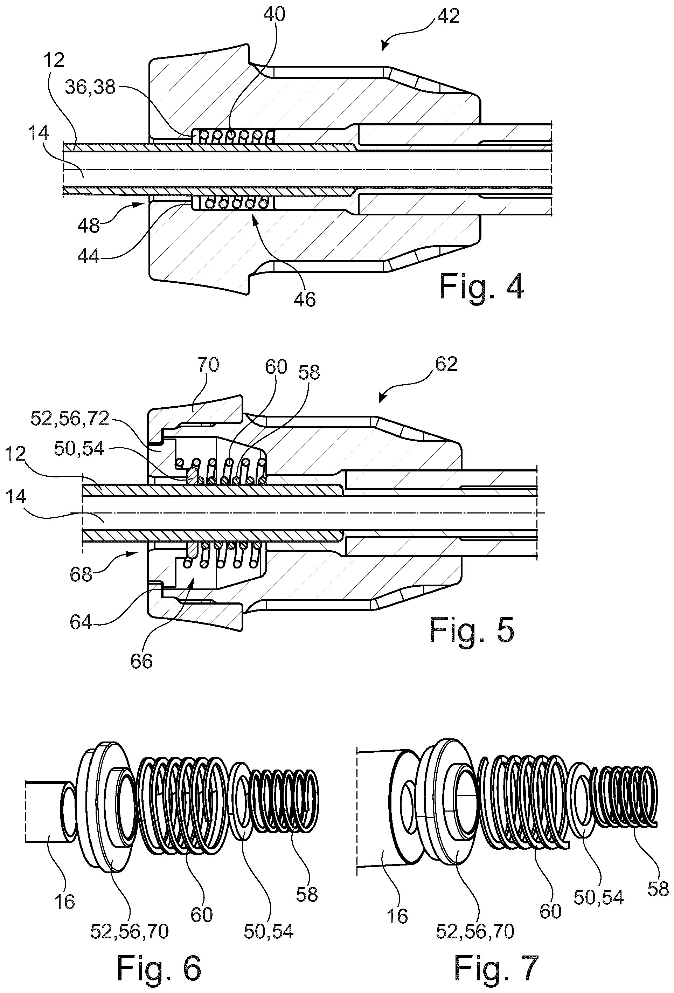

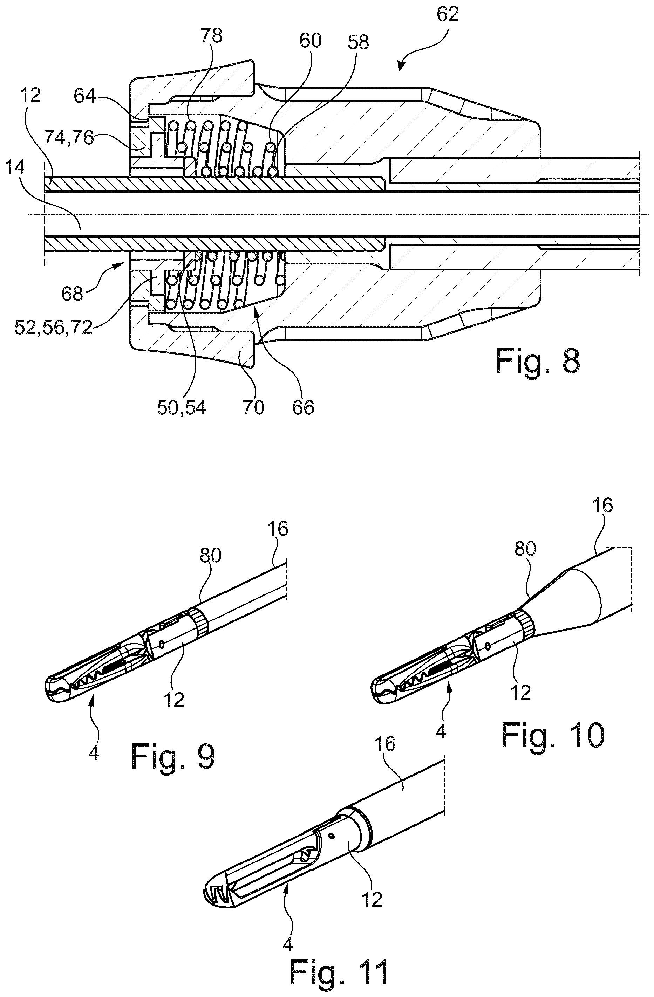

Die Ausbildung bzw. Aufnahme des Isolationsmantels 16 wird mit Bezugnahme auf Fign. 4 bis 11 beschrieben. Fig. 4 zeigt eine Längsschnittdarstellung einer proximalen Aufnahme des Isolationsmantels 16. Fig. 5 zeigt eine Längsschnittdarstellung einer proximalen Aufnahme des Isolationsmantels 16 gemäß einer ersten Ausführungsform. Fign. 6 und 7 zeigen Explosionsdarstellungen von einzelnen Teilen der proximalen Aufnahme des Isolationsmantels 16 gemäß der ersten Ausführungsform. Fig. 8 zeigt eine Längsschnittdarstellung einer proximalen Aufnahme des Isolationsmantels 16 gemäß einer zweiten Ausführungsform. Fign. 9 bis 11 zeigen unterschiedliche Ausführungsformen eines distalen Abschnitts des Isolationsmantels 16 und einer distalen Aufnahme des Isolationsmantels 16.

Der Isolationsmantel 16 ist, wie oben beschrieben, axial verschieblich (/frei schwimmend) auf dem Instrumentenschaft 12 aufgenommen. An dem Instrument 2 ist ein proximaler Axialanschlag 36 zur Begrenzung einer proximal gerichteten Axialbewegung (in Richtung zum Handgriff 8 hin) des Isolationsmantels 16 ausgebildet.

Gemäß einem Aspekt der Offenbarung ist der proximale Axialanschlag 36 axial verschieblich auf dem Instrumentenschaft 12 aufgenommen/angeordnet. Dabei ist der proximale Axialanschlag 36 distal/in eine distale Richtung axialvorgespannt. Das heißt, dass der proximale Axialanschlag 36 eine distal gerichtete Axialkraft auf den Isolationsmantel 16 aufbringt bzw. der proximale Axialanschlag 36 durch eine axiale Vorspannung distal/in die distale Richtung gedrückt wird.

In einem abgekühlten Zustand befindet sich der Isolationsmantel 16 in seiner Montageposition und liegt axial an dem proximalen Axialanschlag 36 an. Durch eine Erwärmung, etwa bei einer Sterilisation des Instruments 2 und/oder der Schaftbaugruppe 6, dehnen sich der Isolationsmantel 16 und der Instrumentenschaft 12, aufgrund ihrer unterschiedlichen Wärmeausdehnungskoeffizienten, unterschiedlich weit aus, so dass es zu einem axialen Verrutschen des Isolationsmantels 16 auf dem Instrumentenschaft 12 kommt. Der proximale Axialanschlag 36 verschiebt sich durch eine Ausdehnung des Isolationsmantels 16 in eine proximale Richtung, wodurch die axiale Vorspannung auf den proximalen Axialanschlag 36 zunimmt. Wenn der Isolationsmantel 16 wieder abkühlt und sich zusammenzieht, wird der Isolationsmantel 16 durch die axiale Vorspannung des proximalen Axialanschlag 36 in seine Montageposition zurückgeschoben.

Fig. 4 zeigt eine Ausbildung der proximalen Aufnahme des Isolationsmantels 16. Der proximale Axialanschlag 36 ist vorzugsweise an einer insbesondere auf dem Instrumentenschaft 12 aufgenommenen (etwa auf einen Außenumfang des Instrumentenschafts 12 aufgesteckten/aufgesetzten) ringförmigen Scheibe 38 ausgebildet. Die axiale Vorspannung des proximalen Axialanschlags 36 ist vorzugsweise durch ein insbesondere auf dem Instrumentenschaft 12 angeordnetes, axial vorspannendes Druckelement, insbesondere eine Feder 40, vorzugsweise in Form einer Schraubenfeder, realisiert. Die Feder 40 kann vorzugsweise direkt an der Scheibe 38 anliegen. Alternativ kann die Feder 40 vorzugsweise direkt an dem Isolationsmantel 16 anliegen, so dass eine axiale Stirnfläche der Feder 40 den proximalen Axialanschlag 36 ausbildet. Der proximale Axialanschlag 36 (d.h. die Scheibe 38 und/oder die Feder 40) kann vorzugsweise innerhalb eines insbesondere auf dem Instrumentenschaft 12 aufgenommenen, kapselartigen Adapters 42 angeordnet sein. Der Adapter 42 kann zur (radial) außenseitigen elektrischen Isolierung des Instrumentenschafts 12 und/oder der Zug-/Druckstange 14 dienen. Der Adapter 42 kann vorzugsweise axialfest mit dem Instrumentenschaft 12 verbunden sein.

Der Adapter 42 kann vorzugsweise einen distalen Anschlag 44 zur Begrenzung einer distal gerichteten Axialbewegung (in Richtung zum Werkzeug 4 hin) des axialverschieblichen proximalen Axialanschlags 36 (d.h. der Scheibe 38 und/oder der Feder 40) aufweisen. Dabei kann eine Axialposition des distalen Anschlags 44 vorzugsweise in Abhängigkeit eines Wärmeausdehungsverhaltens des Isolationsmantels 16 und/oder des Instrumentenschafts 12 festgelegt sein. Insbesondere kann die Axialposition so festgelegt sein, dass der Isolationsmantel 16 in dem abgekühlten Zustand an dem proximalen Axialanschlag 36 (sowie einem später beschriebenen distalen Axialanschlag 80) axial anliegt (d.h., dass sich der Isolationsmantel 16 nicht aufgrund seines Wärmeausdehungsverhaltens weiter in die distale Richtung zurückziehen soll als der proximale Axialanschlag 36 aufgrund des distalen Anschlags 44 in die distale Richtung gedrückt werden kann). Der distale Anschlag 44 kann insbesondere an einem sich radial nach innen (insbesondere weiter über einen Außenumfang des proximalen Axialanschlags 36/der Scheibe 38 hinaus nach innen) erstreckenden Abschnitt des Adapters 42 ausgebildet sein.

Der Adapter 42 kann vorzugsweise eine Aufnahmeschale 46 mit einer Einlegeöffnung zum (axialen) Einlegen (/Einschieben/Einsetzen) des proximalen Axialanschlags 36, insbesondere der Scheibe 38 und/oder der Feder 40, aufweisen. Die Einlegeöffnung kann insbesondere einen größeren Außendurchmesser als der proximale Axialanschlag 36, insbesondere als die Scheibe 38 und/oder die Feder 40, aufweisen. Die Einlegeöffnung kann vorzugsweise auf einer proximalen Seite des Adapters 42 ausgebildet sein. Die Aufnahmeschale 46 kann den distalen Anschlag 44 vorzugsweise direkt/integral ausbilden.

Der Adapter 42 kann vorzugsweise eine distale Öffnung 48 aufweisen, die insbesondere im Wesentlichen so groß ist wie ein Außendurchmesser des Isolationsmantels 16 (bzw. geringfügig größer um die Axialverschiebbarkeit des Isolationsmantels 16 zu gewährleisten), durch die der Isolationsmantel 16 (sowie der Instrumentenschaft 12 und/oder die Zug-/Druckstange 14) axial hindurchgeführt oder hindurchführbar ist. Die distale Öffnung 48 kann (direkt/integral) an einem Innenumfang (/-durchmesser) der Aufnahmeschale 46 ausgebildet sein.

Fign. 5 bis 7 zeigen eine erste Ausführungsform der Ausbildung der proximalen Aufnahme des Isolationsmantels 16. Die proximale Aufnahme gemäß der ersten Ausführungsform kann universell für (zwei) Isolationsmäntel 16 unterschiedlichen Durchmessers dienen. Die erste Ausführungsform unterscheidet sich insbesondere von dem oben beschriebenen Aufbau dadurch, dass zwei proximale Axialanschläge 36 vorhanden sind. Ein erster proximaler Axialanschlag 50 dient zur Begrenzung der Axialbewegung des Isolationsmantels 16 mit einem ersten (kleineren) Durchmesser, während ein zweiter proximale Axialanschlag 52 zur Begrenzung der Axialbewegung des Isolationsmantels 16 mit einem zweiten (größeren) Durchmesser dient, wobei der erste proximale Axialanschlag 50 und der zweite proximale Axialanschlag 52 jeweils im Aufbau im Wesentlichen dem proximalen Axialanschlag 36 des oben beschriebenen Aufbaus entsprechen.

Der erste bzw. zweite proximale Axialanschlag 50, 52 ist vorzugsweise an einer insbesondere auf dem Instrumentenschaft 12 aufgenommenen (etwa auf einen Außenumfang des Instrumentenschafts 12 aufgesteckte/aufgesetzte) ringförmigen ersten Scheibe 54 bzw. zweiten Scheibe 56 ausgebildet. Die axiale Vorspannung des ersten bzw. zweiten proximalen Axialanschlags 50, 52 ist vorzugsweise durch ein insbesondere auf dem Instrumentenschaft 12 angeordnetes, axial vorspannendes Druckelement, insbesondere eine erste Feder 58 bzw. zweite Feder 60, vorzugsweise in Form einer Schraubenfeder, realisiert. Die erste bzw. zweite Feder 58, 60 kann vorzugsweise direkt an der ersten bzw. zweiten Scheibe 54, 56 anliegen. Alternativ kann die erste bzw. zweite Feder 58, 60 vorzugsweise direkt an dem ersten bzw. zweiten Isolationsmantel 16 anliegen, so dass eine axiale Stirnfläche der ersten bzw. zweiten Feder 58, 60 den ersten bzw. zweiten proximalen Axialanschlag 50, 52 ausbildet.

In der proximalen Aufnahme ist insbesondere immer nur ein erster oder zweiter Isolationsmantel 16 eingesetzt. Fig. 6 zeigt eine Explosionsdarstellung (von links nach rechts betrachtet) des Isolationsmantels 16 mit dem ersten (kleineren) Durchmesser, der (den zweiten proximalen Axialanschlag 52 bildenden) zweiten Scheibe 56, der zweiten Feder 60, der ersten Scheibe 54 und der ersten Feder 58. Fig. 7 zeigt eine Explosionsdarstellung (von links nach rechts betrachtet) des Isolationsmantels 16 mit dem zweiten (größeren) Durchmesser, der (den zweiten proximalen Axialanschlag 52 bildenden) zweiten Scheibe 56, der zweiten Feder 60, der ersten Scheibe 54 und der ersten Feder 58.

Vorzugsweise können die erste Feder 58 und die zweite Feder 60 unterschiedliche Federhärten aufweisen. Insbesondere kann die zweite Feder 60 eine größere Federhärte als die erste Feder aufweisen.

Vorzugsweise können die erste Feder 58 und die zweite Feder 60 radial geschachtelt/ineinander angeordnet sein. Insbesondere kann die erste Feder 58 radial innerhalb der zweiten Feder 60 angeordnet sein.

Vorzugsweise können die erste Scheibe 54 und die zweite Scheibe 56 axial aneinander anliegen. Insbesondere kann die erste Scheibe 54 proximal der zweiten Scheibe 56 angeordnet sein.

Vorzugsweise können die erste Scheibe 54 und die zweite Scheibe 56 unterschiedliche Innendurchmesser aufweisen. Insbesondere kann die distal angeordnete Scheibe 54, 56, hier die zweite Scheibe 56, einen größeren Innendurchmesser als die proximal angeordnete Scheibe 54, 56, hier die erste Scheibe 54, aufweisen, so dass der jeweilige Isolationsmantel 16 zum axialen Anliegen an der proximal angeordneten Scheibe 54, 56, hier der ersten Scheibe 54, durch die distal angeordnete Scheibe 54, 56, hier durch die zweite Scheibe 56, axial hindurchführbar ist. Alternativ kann die distal angeordnete Scheibe 54, 56 ein Durchgangsloch aufweisen, durch das der jeweilige Isolationsmantel 16 zum axialen Anliegen an der proximal angeordneten Scheibe 54, 56 hindurchführbar ist.

Der erste bzw. zweite proximale Axialanschlag 50, 52 (d.h. die erste bzw. zweite Scheibe 54, 56 und/oder die erste bzw. zweite Feder 58, 60) kann vorzugsweise innerhalb eines insbesondere auf dem Instrumentenschaft 12 aufgenommenen, kapselartigen Adapters 62 angeordnet sein. Der Adapter 62 kann zur (radial) außenseitigen elektrischen Isolierung des Instrumentenschafts 12 und/oder der Zug-/Druckstange 14 dienen. Der Adapter 62 kann vorzugsweise axialfest mit dem Instrumentenschaft 12 verbunden sein.

Der Adapter 62 kann vorzugsweise einen distalen Anschlag 64 zur Begrenzung einer distal gerichteten Axialbewegung (in Richtung zum Werkzeug 4 hin) des axialverschieblichen ersten bzw. zweiten proximalen Axialanschlags 50, 52 aufweisen. Dabei kann eine Axialposition des distalen Anschlags 64 vorzugsweise in Abhängigkeit eines Wärmeausdehungsverhaltens des Isolationsmantels 16 und/oder des Instrumentenschafts 12 festgelegt sein. Insbesondere kann die Axialposition so festgelegt sein, dass der Isolationsmantel 16 in dem abgekühlten Zustand an dem ersten bzw. zweiten proximalen Axialanschlag 50, 52 (sowie einem später beschriebenen distalen Axialanschlag 80) axial anliegt (d.h., dass sich der Isolationsmantel 16 nicht aufgrund seines Wärmeausdehungsverhaltens weiter in die distale Richtung zurückziehen soll als der erste bzw. zweite proximale Axialanschlag 50, 52 aufgrund des distalen Anschlags 64 in die distale Richtung gedrückt werden kann). Der distale Anschlag 64 kann insbesondere an einem sich radial nach innen (insbesondere weiter über einen Außenumfang des ersten bzw. zweiten proximalen Axialanschlags 50, 52 hinaus nach innen) erstreckenden Abschnitt des Adapters 62 ausgebildet sein.

Der Adapter 62 kann vorzugsweise eine Aufnahmeschale 66 mit einer Einlegeöffnung zum (axialen) Einlegen (/Einschieben/Einsetzen) des ersten bzw. zweiten proximalen Axialanschlags 50, 52, insbesondere der ersten bzw. zweiten Scheibe 54, 56 und/oder der ersten bzw. zweiten Feder 58, 60, aufweisen. Die Einlegeöffnung kann insbesondere einen größeren Außendurchmesser als der erste bzw. zweite proximale Axialanschlag 50, 52, insbesondere als die erste bzw. zweite Scheibe 54, 56 und/oder die erste bzw. zweite Feder 58, 60, aufweisen. Die Einlegeöffnung kann vorzugsweise auf einer distalen Seite des Adapters 62 ausgebildet sein.

Der Adapter 62 kann vorzugsweise eine distale Öffnung 68 aufweisen, deren Außendurchmesser insbesondere im Wesentlichen dem des Isolationsmantels 16 (bzw. geringfügig größer ist um die Axialverschiebbarkeit des Isolationsmantels 16 zu gewährleisten), durch die der Isolationsmantel 16 (sowie der Instrumentenschaft 12 und/oder die Zug-/Druckstange 14) axial hindurchgeführt oder hindurchführbar ist. Alternativ kann die distale Öffnung 68 im Wesentlichen so groß wie ein Außendurchmesser des Instrumentenschafts 12 sein, wenn der Isolationsmantel 16 zur Kontaktierung der distal angeordneten Scheibe 54, 56, hier der ersten Scheibe 54, durch das Durchgangsloch hindurchgreift.

Der Adapter 62 eine vorzugsweise separat von der Aufnahmeschale 66 ausgebildete Kappe 70 aufweisen, die von distaler Seite auf die Aufnahmeschale 66 aufsteckbar, insbesondere aufschraubbar ist. Die Kappe 70 kann den distalen Anschlag 64 vorzugsweise direkt/integral ausbilden. Der Adapter 62 kann vorzugsweise einen Axialdeckel 72 aufweisen, der die Einlegeöffnung an der Aufnahmeschale 66 verschließt und die distale Öffnung 68 an seinem Innenumfang (/-durchmesser) (direkt/integral) ausbildet. Der Axialdeckel 72 kann vorzugsweise durch die erste Scheibe 54 oder die zweite Scheibe 56 gebildet sein, insbesondere durch die proximal angeordnete Scheibe 54, 56, hier die zweite Scheibe 56. Alternativ kann die distale Öffnung 68 (direkt/integral) an einem Innenumfang (/-durchmesser) der Kappe 70 ausgebildet sein, wenn der Isolationsmantel 16 zur Kontaktierung der distal angeordneten Scheibe 54, 56, hier der ersten Scheibe 54, durch ein Durchgangsloch in der Kappe 70 hindurchgreift.

Fig. 8 zeigt eine zweite Ausführungsform der Ausbildung der proximalen Aufnahme des Isolationsmantels 16. Die proximale Aufnahme gemäß der zweiten Ausführungsform kann universell für (drei) Isolationsmäntel 16 unterschiedlichen Durchmessers dienen. Der Aufbau der proximalen Aufnahme gemäß der zweiten Ausführungsform entspricht im Wesentlichen dem der ersten Ausführungsform. Zusätzlich ist ein dritter proximaler Axialanschlag 74 vorgesehen, der durch eine dritte Scheibe 76 gebildet ist und über eine dritte Feder 78 in eine distale Richtung axialvorgespannt ist. Die drei Scheiben 54, 56, 76 liegen axial aneinander an. Die drei Federn 58, 60, 78 sind radial geschachtelt angeordnet.

Fign. 9 bis 11 zeigen unterschiedliche Ausführungsformen eines distalen Abschnitts des Isolationsmantels 16 und einer distalen Aufnahme des Isolationsmantels 16.

An dem Instrument 2, insbesondere an dem Instrumentenschaft 12 (oder einem damit (translationsfest) verbundenen Bauteil), ist insbesondere ein distaler Axialanschlag 80 zur Begrenzung einer distal gerichteten Axialbewegung (in Richtung zum Werkzeug 4 hin) des Isolationsmantels 16 ausgebildet. Der distale Axialanschlag 80 kann axialfest zu dem Instrumentenschaft 12 aufgenommen sein, insbesondere axialfest mit dem Instrumentenschaft 12 verbunden bzw. an dem Instrumentenschaft 12 ausgebildet sein. In Fig. 9 liegt der Isolationsmantel 16 mit dem ersten (kleineren) Durchmesser, in Fig. 10 liegt der Isolationsmantel 16, dessen Durchmesser sich an seinem distalen Ende von dem zweiten (größeren) Durchmesser auf den ersten (kleineren) Durchmesser verjüngt, und in Fig. 11 liegt der Isolationsmantel 16 mit dem zweiten (größeren) Durchmesser an dem distalen Axialanschlag 80 an.

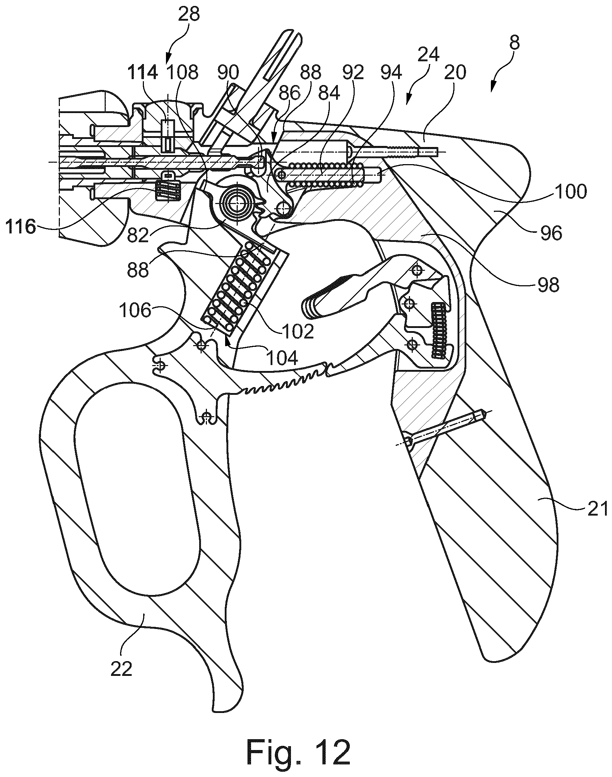

Fig. 12 zeigt eine Längsschnittdarstellung des Handgriffs 8, anhand der die Ausbildung des Getriebes 24 näher erläutert wird. Wie obenstehend beschrieben, ist das Getriebe 24 ausgebildet, um die Schwenkbewegung des Betätigungshebels 22 in die Translationsbewegung der Transmission, insbesondere der Längsbewegung der Zug-Druckstange 14 zu übertragen. Das heißt, dass das Getriebe 24 die (durch die manuelle Betätigung aktuierte) Schwenkbewegung des Betätigungshebels 22 mit der Längsbewegung der Zug-/Druckstange 14 koppelt (und damit (indirekt) mit dem Betätigen des Werkzeugs 4 bzw. der Schwenkbewegung der Werkzeugbranchen 10).

Dabei ist die Betätigungshebelschwenkachse, um die der Betätigungshebel 22 an dem Getriebegehäuse 20 drehbar angelenkt ist, insbesondere zwischen der Translationsachse der Translationsbewegung der Transmission, insbesondere der Schaftachse (d.h. der Längsachse des Instrumentenschafts 12 bzw. der Zug-/Druckstange 14), und einem proximalen Endbereich des Betätigungshebels 22 (d.h. der Betätigungskraft-Angriffsstelle zum Aufbringen der Betätigungskraft) angeordnet. Mit anderen Worten befindet sich die Betätigungshebelschwenkachse (bei Benutzung des Instruments 2 in Vertikalrichtung) unterhalb der Schaftachse.

Gemäß einem Aspekt der vorliegenden Offenbarung ist das Getriebe 24 so ausgebildet ist, dass eine Schwenkbewegung des Betätigungshebels 22 hin zum Griffelement 21 in eine Translationsbewegung der Transmission 14 in Richtung proximal transformiert wird. Das heißt, dass die Schwenkbewegung des Betätigungshebels 22 hin zum Griffelement 21 (das Betätigen des Betätigungshebels 22) in eine proximal gerichtete Längsbewegung der Zug-/Druckstange 14 (eine Zugbewegung der Zug-/Druckstange 14) gewandelt wird.

Insbesondere ist das Getriebe 24 dazu zweiteilig oder mehrteilig ausgebildet. Vorzugsweise bildet das Getriebe 24 einen Kraftübertragungszug (vom Betätigungshebel 22 zur Transmission (Zug-/Druckstange 14)).

Der Kraftübertragungszug weist ein erstes Drehteil (/Stellhebel) 82 auf. Das erste Drehteil 82 steht mit dem Betätigungshebel 22 in Wirkeingriff. Das heißt, dass die Schwenkbewegung des Betätigungshebels 22 mit einer Rotation des erstes Drehteils 82 gekoppelt ist. Das erste Drehteil 82 ist an dem Getriebegehäuse 20 um eine erste Drehachse drehbar angelenkt. Die erste Drehachse ist insbesondere quer bzw. senkrecht zu der Schaftachse. Das erste Drehteil 82 ist insbesondere separat zu dem Betätigungshebel 22 ausgebildet, kann aber alternativ auch an diesem (d.h. an einem Abschnitt des Betätigungshebels 22) ausgebildet sein, auch wenn dies nicht dargestellt ist.

Der Kraftübertragungszug weist ein zweites Drehteil (/Aufnahmeelement/Schließelement) 84 auf. Das zweite Drehteil 84 steht mit dem ersten Drehteil 82 unter Drehrichtungsumkehr in Wirkeingriff, vorzugsweise in Verzahnungseingriff. Das heißt, dass die Rotation des ersten Drehteils 82 mit einer Rotation des zweiten Drehteils 84 gekoppelt ist, und das erste Drehteil 82 und das zweite Drehteil 84 in unterschiedliche Drehrichtungen rotieren. Das zweite Drehteil 84 ist an dem Getriebegehäuse 20 um eine zweite Drehachse drehbar angelenkt. Die zweite Drehachse ist insbesondere quer bzw. senkrecht zu der Schaftachse. Die zweite Drehachse ist vorzugsweise parallel versetzt zu der ersten Drehachse. Das zweite Drehteil 84 hat vorzugsweise einen Kopplungsabschnitt 86 für die Transmission (/ Zug-/Druckstange 14). Der Kopplungsabschnitt 86 ist insbesondere mit der Transmission (/Zug-/Druckstange 14) in Wirkeingriff stehend oder bringbar. Das heißt, dass die Rotation des zweiten Drehteils 84 über den Kopplungsabschnitt 86 mit der Translationsbewegung der Transmission, insbesondere der Längsbewegung der Zug-/Druckstange 14, gekoppelt oder koppelbar ist.

Dazu können das erste Drehteil 82 und das zweite Drehteil 84 miteinander in Verzahnungseingriff stehende Zähne aufweisen. Die Verzahnung kann beispielsweise als eine Evolventenverzahnung ausgebildet sein. Durch den Verzahnungseingriff rotieren das erste Drehteil 82 und das zweite Drehteil 84 in unterschiedliche Drehrichtungen. Dies hat zur Folge, dass die Schwenkbewegung des Betätigungshebels 22 in der Betätigungsrichtung die Längsbewegung der Zug-/Druckstange 14 in die Zugrichtung und die Schwenkbewegung des Betätigungshebels 22 in der Rückstellrichtung die Längsbewegung der Zug-/Druckstange 14 in die Schubrichtung hervorruft (/erzwingt/aktuiert).

Vorzugsweise kann der Instrumentenschaft 12 in gekoppeltem Zustand als Anschlag für die Schwenkbewegung des Betätigungshebels 22, d.h. als Aufschwenkbegrenzung für den Betätigungshebel 22, bzw. als Rotationsbegrenzung für das erste Drehteil 82 und das zweite Drehteil 84 bzw. als Translationsbegrenzung für die Transmission, insbesondere als Längsbegrenzung für die Zug-/Druckstange 14 dienen. Das heißt, dass der Betätigungshebel 22 (nur) innerhalb eines, vorzugsweise beidseitig begrenzten Schwenkbereichs verschwenkbar ist bzw. das erste Drehteil 82 und das zweite Drehteil 84 (nur) innerhalb eines, vorzugsweise beidseitig begrenzten Rotationsbereichs bzw. die Transmission (/Zug-/Druckstange 14) (nur) innerhalb eines, vorzugsweise beidseitig begrenzten Translationsbereichs (/Längsbereich/Arbeitsbereich) längsbeweglich ist, d.h. einen maximalen Hub der Längsbewegung hat. Durch die Kopplung zwischen der Transmission (/Zug-/Druckstange 14) und dem Getriebe 24 (insbesondere dem zweiten Drehteil 82), der Kraftübertragung innerhalb des Getriebes 24 (insbesondere dem zweiten Drehteil 82 und dem ersten Drehteil 82) sowie der Kopplung zwischen dem Getriebe 24 (insbesondere dem ersten Drehteil 82) und dem Betätigungshebel 22 kann der Anschlag am Instrumentenschaft 12 für alle miteinander gekoppelten (oder koppelbaren) Bewegungen dienen.

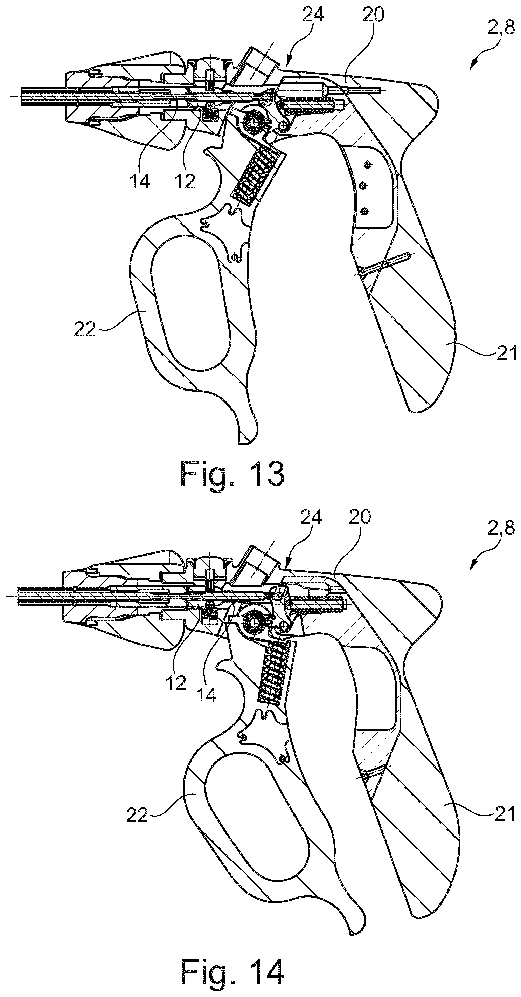

Fign. 13 und 14 zeigen Endstellungen des Translationsbereichs/Arbeitsbereichs der Transmission, in der das Werkzeug 4 vollständig geöffnet bzw. geschlossen ist.

Vorzugsweise kann das Getriebe 24 ein Übersetzungsverhältnis von 1:1 aufweisen. Insbesondere können die Zähne des ersten Drehteils 82 und des zweiten Drehteils 84 auf demselben Durchmesser angeordnet sein. Alternativ könnten die Zähne des ersten Drehteils 82 und des zweiten Drehteils 84 auf unterschiedlichen Durchmessern angeordnet sein, um eine Untersetzung oder eine Übersetzung zu realisieren, auch wenn es nicht dargestellt ist.

Vorzugsweise können die Zähne des ersten Drehteils 82 und/oder die Zähne des zweiten Drehteils 84 (nur) umfangsseitig abschnittsweise, d.h. nicht über einen gesamten Umfang, ausgebildet sein. Dabei kann eine Anzahl der Zähne des ersten Drehteils 82 und/oder des zweiten Drehteils 84, d.h. eine Dimensionierung der umfangsseitig abschnittsweisen Ausbildung, vorzugsweise in Abhängigkeit des maximalen Hubs der Längsbewegung/des begrenzten Längsbereichs der Zug-/Druckstange 14 festgelegt sein. Insbesondere können das erste Drehteil 82 und/oder das zweite Drehteil 84 zwei bis fünf Zähne, vorzugsweise zwei, drei oder vier Zähne, aufweisen. Alternativ könnten die Zähne des ersten Drehteils 82 und/oder des zweiten Drehteils 84 über den gesamten Umfang ausgebildet sein, auch wenn dies nicht dargestellt ist.

Vorzugsweise kann der Kopplungsabschnitt 86 des zweiten Drehteils 84 als eine axial hinterschnittene Ausnehmung 88 ausgebildet sein, in welche die Transmission (Zug-/Druckstange 14) axial hinterschneidend eingreifen kann oder eingreift, um die Drehung des zweiten Drehteils 84 mit der Translationsbewegung (/Längsbewegung der Zug-/Druckstange 14) zu koppeln. Dazu kann die Zug-/Druckstange 14 an ihrem proximalen Ende (Endbereich) eine, insbesondere in Form eines Kugeldruckstücks 90 ausgebildete, radiale (gegenüber einem axial angrenzenden Bereich vergrößerte) Verdickung aufweisen, die in die Ausnehmung 88 des zweiten Drehteils 84 axial hinterschneidend eingreift.

Vorzugsweise kann das Getriebe 24 einen an dem zweite Drehteil 84 (drehbar) angelenkten Führungsdorn 92 aufweisen. Der Führungsdorn 92 ist insbesondere an dem zweiten Drehteil 84 um eine Dorndrehachse drehbar angelenkt, so dass das zweite Drehteil 84 und der Führungsdorn 92 relativ zueinander verdreht werden können. Die Dorndrehachse ist insbesondere quer bzw. senkrecht zu der Schaftachse. Vorzugsweise kann die Dorndrehachse parallelversetzt zu der zweiten Drehachse (und/oder der ersten Drehachse) sein. Der Führungsdorn 92 ist längsgeführt, d.h. (nur) entlang seiner Längsachse verschieblich, in dem Getriebegehäuse 20 aufgenommen. Dabei kann die Längsachse des Führungsdorns 92 insbesondere parallelversetzt zu der Schaftachse sein. Vorzugsweise ist der Führungsdorn 92, über eine Feder 94, federvorgespannt in dem Getriebegehäuse 20 aufgenommen. Vorzugsweise kann die Federvorspannung des Führungsdorns 92 der Schwenkbewegung des Betätigungshebels 22 hin zum Griffelement 21 (d.h. der Betätigung) entgegenwirken.

Vorzugsweise kann der Handgriff 8 einen Außengriff 96 und einen fest mit dem Außengriff 96 verbundenen, beispielsweise angeschraubten Innengriff 98 aufweisen. Vorzugsweise kann der Innengriff 98 von dem Außengriff 96 außenseitig abgedeckt sein. Insbesondere kann das zweite Drehteil 84 drehbar an dem Innengriff 98 angelenkt sein. Insbesondere kann der Führungsdorn 92 drehbar an dem Innengriff 98 angelenkt sein. Zudem kann der Führungsdorn 92 in einer Aussparung 100 in dem Innengriff 98 längsgeführt aufgenommen sein.

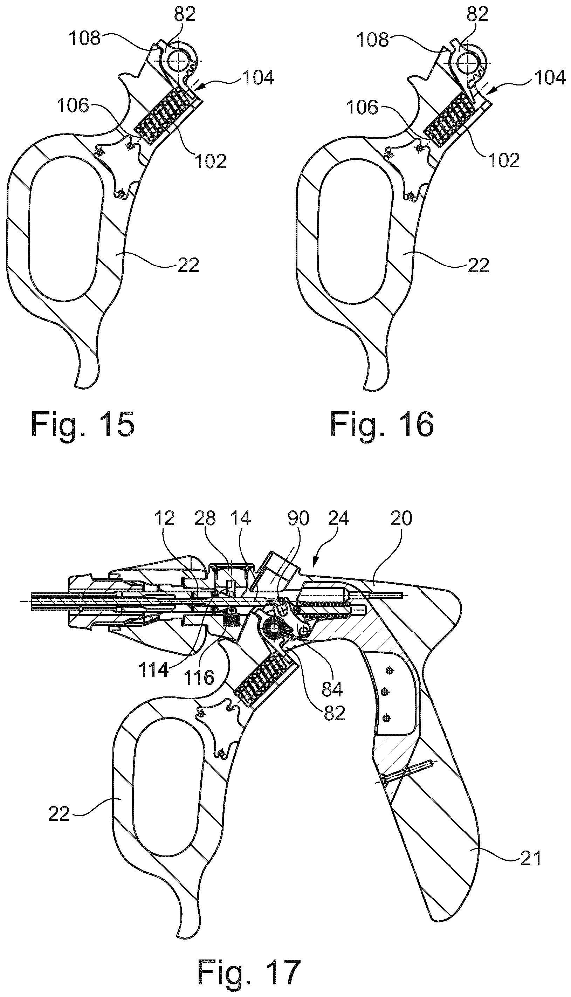

Gemäß einem Aspekt der vorliegenden Offenbarung weist das Getriebe 24 das insbesondere an dem Getriebegehäuse 20 (drehbar) angelenkte erste Drehteil 82 sowie ein die Schwenkbewegung des Betätigungshebels 22 mit einer Rotation des ersten Drehteils 82 koppelndes, elastisches Überlastsicherungselement 102 auf (vgl. insbesondere auch Fign. 15 und 16). Das heißt, dass das Überlastsicherungselement 102 im Kraftfluss/Kraftübertragungszug zwischen dem Betätigungshebel 22 und dem ersten Drehteil 82 angeordnet ist. Das Überlastsicherungselement 102 kann insbesondere als ein Federelement, vorzugsweise als eine Schraubenfeder ausgebildet sein.

Vorzugsweise können der Betätigungshebel 22 und das erste Drehteil 82 um dieselbe Achse schwenkbar bzw. drehbar an dem Getriebegehäuse 20 angelenkt sein. Das heißt, dass die Betätigungshebelschwenkachse vorzugsweise der ersten Drehachse entspricht.

Vorzugsweise kann der Betätigungshebel 22 eine Ausnehmung 104 aufweisen, in der das Überlastsicherungselement 102 aufgenommen ist. Insbesondere kann das Überlastsicherungselement 102 vollständig innerhalb der Ausnehmung 104 angeordnet sein. Das heißt, dass das Überlastsicherungselement 102 vorzugsweise nach außen hin durch den Betätigungshebel 22 abgedeckt ist.

Vorzugsweise kann das Überlastsicherungselement 102 lose in der Ausnehmung 104 aufgenommen sein, d.h. nicht fest mit dem Betätigungshebel 22 und/oder dem ersten Drehteil 82 verbunden sein. Insbesondere kann das Überlastsicherungselement 102 längsgeführt, d.h. d.h. (nur) entlang seiner Längsachse verschieblich bzw. elastisch komprimierbar bzw. verbiegbar in der Ausnehmung 104 aufgenommen sein.

Wie oben beschrieben, kann der Betätigungshebel 22 in die Betätigungsrichtung, d.h. in die Richtung von dem Betätigungshebel 22 aus zu dem Griffelement 21 hin, aktuiert etwa durch Schließen einer Hand des Operateurs/Zusammendrücken von Betätigungshebel 22 und Griffelement 21, und in die Rückstellrichtung, d.h. in die Richtung von dem Betätigungshebel 22 aus von dem Griffelement 21 weg, aktuiert etwa durch Öffnen einer Hand des Operateurs/Auseinanderdrücken von Betätigungshebel 22 und Griffelement 21, verschwenkt werden, d.h. betätigt und rückgestellt werden.

Dabei kann das Überlastsicherungselement 102 vorzugsweise so angeordnet sein, dass es (nur/ausschließlich) in der Betätigungsrichtung (und nicht in der Rückstellrichtung) wirkt. Das heißt, dass das Überlastsicherungselement 102 nur die Schwenkbewegung des Betätigungshebels 22 zum Griffelement 21 hin begrenzt/dämpft/vor Überlast sichert/schützt. Insbesondere können eine Betätigungsübertragungsfläche 106 des Betätigungshebels 22 und eine Rückstellübertragungsfläche 108 des Betätigungshebels 22 voneinander getrennt, d.h. an unterschiedlichen Flächen, ausgebildet sein. Das heißt, dass der Betätigungshebel 22 und das erste Drehteil 82 nicht fest miteinander verbunden sind, und eine Kraftübertragung bei dem Betätigen des Betätigungshebels 22 über die über das Überlastsicherungselement 102 mit dem ersten Drehteil 82 gekoppelte Betätigungsübertragungsfläche 106 und eine Kraftübertragung bei dem Rückstellen des Betätigungshebels 22 (direkt) über die an dem ersten Drehteil 82 anliegende gekoppelte Rückstellübertragungsfläche 108 erfolgt.

Zudem kann das Überlastsicherungselement 102 vorzugsweise so angeordnet und dimensioniert sein, dass eine anfängliche (anfänglich im Sinne der Betätigung, d.h. ausgehend von der Schwenkbewegung aus einer unbetätigten Stellung des Betätigungshebels 22) Kraftübertragung über das Überlastsicherungselement 102 zwischen dem Betätigungshebel 22 und dem ersten Drehteil 82 ein im Wesentlichen lineares Übertragungsverhalten aufweist. Das heißt, dass die Kopplung über das Überlastsicherungselement 102 bei der anfänglichen Kraftübertragung, insbesondere bei Kraftübertragung innerhalb des "normalen" Arbeitsbereichs (und ohne Widerstand am Werkzeug 4), quasi starr/unelastisch ist.

Insbesondere kann das Überlastsicherungselement 102 vorzugsweise so angeordnet und dimensioniert sein, dass das Überlastsicherungselement erst ab einer über den Betätigungshebel 22 aufgebrachten Betätigungskraft größer 200 N, vorzugsweise größer 300 N, (aber maximal erst ab einer über den Betätigungshebel 22 aufgebrachten Betätigungskraft von 1000 N) komprimiert wird.

Gemäß einem Aspekt der vorliegenden Offenbarung kann der Instrumentenschaft 12 an den Handgriff 8 gekoppelt oder koppelbar sein und in gekoppeltem Zustand als Anschlag für die Schwenkbewegung des Betätigungshebels 22 (und der im Kraftübertragungszug damit gekoppelten Bauteile) dienen. Das heißt, dass der Instrumentenschaft 12 im gekoppelten Zustand als Aufschwenkbegrenzung für den Betätigungshebel 22, sowie als Translationsbegrenzung für die Transmission bzw. Längsbegrenzung für die Zug-/Druckstange 14, und damit als Wegbegrenzung für das Werkzeug 4 dient. Das heißt auch, dass in einem entkoppelten Zustand des Instrumentenschafts 12 die Anschlagsfunktion für die Schwenkbewegung des Betätigungshebels 22 sowie die Translationsbegrenzung für die Transmission bzw. die Längsbegrenzung für die Zug-/Druckstange 14, und damit die Wegbegrenzung für das Werkzeug 4 entfällt, so dass die beweglich geführten oder angelenkten Bestandteile des Kraftübertragungszugs des Getriebes 24 bzw. des Instruments 2 sich frei (bezüglich ihres Freiheitsgrads/ihrer aufgrund einer entsprechenden Lagerung/Aufnahme bzw. Anbringung möglichen Bewegung) bewegen können.

Dabei kann der Instrumentenschaft 12 derart als Anschlag dienen, dass eine Entkopplung der Transmission (/Zug-/Druckstange 14) von dem Getriebe 24 nur im entkoppelten Zustand des Instrumentenschafts 12 vom Handgriff 8 infolge des Wegfalls seiner Anschlagfunktion möglich ist. Das heißt, dass eine Entkopplung der Transmission (/Zug-/Druckstange 14) von dem Getriebe 24 im am Handgriff 8 gekoppelten Zustand des Instrumentenschafts 12 infolge dessen Anschlagsfunktion gesperrt ist.

Wie oben beschrieben, kann der durch den Instrumentenschaft 12 gebildete Anschlag vorzugsweise als ein die Translationsbewegung der Transmission (/die Längsbewegung der Zug-/Druckstange 14) insbesondere beidseitig begrenzender Axialanschlag ausgebildet sein.

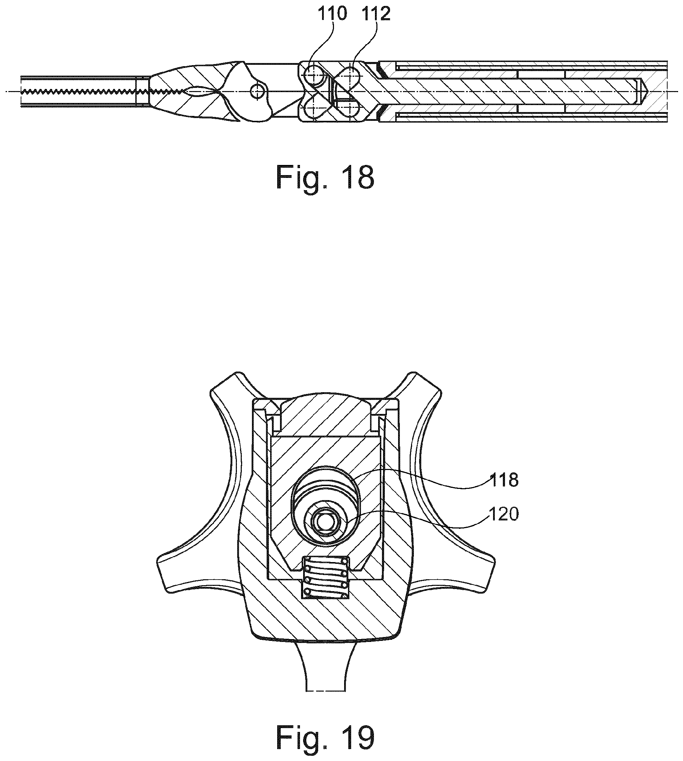

Beispielsweise kann der Anschlag dadurch gebildet sein, dass die Transmission (/Zug-/Druckstange 14) einen Zapfen 110 hat, der in einer Kulisse 112 an dem Instrumentenschaft 12 aufgenommen ist, so dass bei am Kulissenrand anliegendem Zapfen 110 eine weitere Translationsbewegung (in Richtung des Kulissenrands) unterbunden ist (vgl. Fig. 18). Vorzugsweise kann der Anschlag an einem distalen Endbereich des Instrumentenschaft 12 ausgebildet sein.

Vorzugsweise kann das Getriebe 24 das mit dem Betätigungshebel 22 in Wirkverbindung stehende zweite Drehteil 84 aufweisen, dessen Rotation über eine (erste) axiale Formschlussverbindung mit der Translationsbewegung der Transmission (/der Längsbewegung der Zug-/Druckstange 14) koppelbar oder gekoppelt ist. Die erste axiale Formschlussverbindung ist insbesondere durch die axial hinterschnittene Ausnehmung 88 im zweiten Drehteil 84 ausgebildet, in die die Transmission, insbesondere das Kugeldruckstück 90 der Zug-/Druckstange 14 (im gekoppelten Zustand des Instrumentenschafts 12 und damit der Zug-/Druckstange 14) axial hinterschneidend eingreift.

Vorzugsweise kann die Transmission (/Zug-Druckstange 14) im entkoppelten Zustand des Instrumentenschafts 12 so weit translatorisch verschiebbar, insbesondere längsbeweglich, sein und (aufgrund der Kopplung zwischen der Transmission und dem zweiten Drehteil 84 bzw. dem Getriebe 24) kann insbesondere das zweite Drehteil 84 im entkoppelten Zustand des Instrumentenschafts (12) so weit drehbar sein, dass die axiale Formschlussverbindung zwischen der Transmission und dem zweiten Drehteil 84 lösbar ist. Diese Stellung des zweiten Drehteils 84 wird im Folgenden auch als eine Demontagestellung des zweiten Drehteils 84 bezeichnet bzw. als Ladeposition des Instruments 2 bezeichnet (vgl. insbesondere Fig. 17). Insbesondere kann die Transmission (/Zug-/Druckstange 14) (zusammen mit dem Instrumentenschaft 12 bzw. der gesamten Schaftbaugruppe 6) in die distale Richtung (aus dem Handgriff 8 heraus) gezogen werden.

Zudem kann das zweite Drehteil 84 federvorgespannt in dem Handgriff 8, insbesondere dem Getriebegehäuse 20, aufgenommen sein. Dabei kann insbesondere eine Federvorspannung des zweiten Drehteils 84 das zweite Drehteil 84 in die Demontagestellung drücken, in der die erste axiale Formschlussverbindung zwischen der Transmission (/Zug-/Druckstange 14) und dem zweiten Drehteil 84 lösbar ist. Die Federvorspannung kann insbesondere über die den Führungsdorn 92 vorspannende Feder 94 realisiert sein. Die Demontagestellung kann insbesondere einer unbetätigt überstreckten Stellung des Betätigungshebels 22 entsprechen, d.h. einer Stellung, in der der Betätigungshebel 22 weiter von dem Griffelement 21 weg positioniert ist als in der unbetätigten Stellung.

Insbesondere kann der Instrumentenschaft 12 im gekoppelten Zustand axialfest mit dem Handgriff 8 gekoppelt sein und im entkoppelten Zustand axialverschieblich zu dem Handgriff 8 sein. Das heißt, dass eine Kopplung zwischen dem Instrumentenschaft 12 und dem Handgriff 8 insbesondere einer axialfesten Verbindung entspricht.

Wie oben beschrieben kann das Instrument 2 bzw. der Handgriff 8 vorzugsweise den Demontageknopf 28 aufweisen, bei dessen Betätigung (/Drücken) die Schaftbaugruppe 6/der Instrumentenschaft 12 und der Handgriff 8 demontierbar sind, d.h. die Schaftbaugruppe 6/der Instrumentenschaft 12 aus dem Handgriff 8 auskoppelbar ist. Der Demontageknopf 28 kann insbesondere manuell betätigbar sein. Durch ein Betätigen des Demontageknopfs 28 kann der Instrumentenschaft 12 von dem Handgriff 8 axial entkoppelbar sein. Das heißt, dass der Demontageknopf 28 eine Axialverschiebung des Instrumentenschafts 12 relativ zum Handgriff 8 sperren kann. Mit anderen Worten können der Instrumentenschaft 12 und der Handgriff bei betätigtem Demontageknopf 28 zueinander frei axial verschoben werden und bei unbetätigtem Demontageknopf 28 axial miteinander verbunden werden bzw. sein.

Vorzugsweise kann der Demontageknopf 28 einen (längs-)verschieblich in dem Handgriff 8 aufgenommenen Schließschieber 114 aufweisen, dessen Längsachse insbesondere einer Radialrichtung des Instrumentenschafts 12 entspricht. Das heißt, dass der Schließschieber 114 quer bzw. senkrecht zu der Schaftachse verschieblich ist. Der Schließschieber 114 kann vorzugsweise über eine zweite axiale Formschlussverbindung mit dem Instrumentenschaft 12 koppelbar oder gekoppelt sein. Dabei kann die zweite axiale Formschlussverbindung vorzugsweise durch eine (Längs-)Verschiebung des Demontageknopfs 28 (relativ zu dem Handgriff 8) lösbar sein.

Insbesondere kann der Schließschieber 114 federvorgespannt in dem Handgriff aufgenommen sein. Dabei kann die (zweite) axiale Formschlussverbindung vorzugsweise entgegen einer Federvorspannung des Schließschiebers 114 lösbar sein. Das heißt, dass eine Federvorspannung einer Feder 116 den Schließschieber 114 in eine unbetätigte Position drückt bzw. in axial formschlüssigen Eingriff mit dem Instrumentenschaft 12 drückt.

Beispielsweise kann die zweite axiale Formschlussverbindung durch eine in dem Schließschieber 114 ausgebildete langlochartige Kulisse 118 gebildet sein, mit welcher der Instrumentenschaft 12 (bei betätigtem Demontageknopf 28) außer axial formschlüssigem Eingriff ist und somit axial durch die Kulisse 118 hindurchführbar ist und (bei unbetätigtem Demontageknopf 28) in axial formschlüssigen Eingriff ist und somit nicht axial zu der Kulisse 118 (und damit zum Handgriff 8) bewegbar ist. Dazu kann der Instrumentenschaft 12 vorzugsweise eine (etwa umfangsseitig umlaufende) Radialnut 120 aufweisen, in die ein Endbereich/Kulissenrand der Kulisse 118 in der unbetätigten Position des Demontageknopfs 28 eingreift (und dadurch eine Axialbewegung des Instrumentenschafts 12 (in Richtung durch die Kulisse hindurch) unterbindet) und in einer betätigten Position (/längsverschobenen Position) des Demontageknopfs 28 in einem zentralen Bereich/einer Kulissenmitte der Kulisse 118 aufgenommen ist (vgl. Fig. 19).

## Patentansprüche

1. Chirurgische Schaftbaugruppe (6) eines oder für ein chirurgisches Instrument (2), insbesondere elektrochirurgisches Instrument (2) der minimalinvasiven Schaftbauart, an deren distalen Ende ein Werkzeug (4) angekoppelt oder ankoppelbar ist und an deren proximalen Ende ein Handgriff (8), insbesondere nach Art eines Pistolen-Handgriffs zur Betätigung des Werkzeugs (4) ankoppelbar ist, mit
- einem insbesondere aus einem Metall, vorzugsweise aus Stahl, ausgebildeten Instrumentenschaft (12);
- einem aus einem zum Instrumentenschaft (12) unterschiedlichen Material, insbesondere aus Kunststoff, vorzugsweise aus PEEK, ausgebildeten Isolationsmantel (16), der axial verschieblich auf dem Instrumentenschaft (12) angeordnet ist und den Instrumentenschaft (12) radial außenseitig zur elektrischen Isolierung des Instrumentenschafts (12), vorzugsweise umfangsseitig vollständig, ummantelt, und
- einen axial verschieblich auf dem Instrumentenschaft (12) aufgenommenen und in eine distale Richtung axialvorgespannten, proximalen Axialanschlag (36, 50, 52, 74) zur Begrenzung einer proximal gerichteten Axialbewegung des Isolationsmantels (16) relativ zu dem Instrumentenschaft (12),
**dadurch gekennzeichnet, dass** die Schaftbaugruppe (6) zwei der proximalen Axialanschläge (50, 52) aufweist, von denen ein erster proximaler Axialanschlag (50) zur Begrenzung der Axialbewegung des Isolationsmantels (16) mit einem ersten Durchmesser und ein zweiter proximaler Axialanschlag (52) zur Begrenzung der Axialbewegung des Isolationsmantels (16) mit einem zweiten Durchmesser dient.

2. Schaftbaugruppe (6) nach Anspruch 1, **gekennzeichnet durch** eine vorzugsweise ringförmige, den proximalen Axialanschlag (36, 50, 52, 74) ausbildende Scheibe (38, 54, 56, 76).

3. Schaftbaugruppe (6) nach Anspruch 1 oder 2, **gekennzeichnet durch** ein den proximalen Axialanschlag (36, 50, 52, 74) axial vorspannendes Druckelement (40, 58, 60, 78), insbesondere in Form einer Feder, vorzugsweise Schraubenfeder, welches vorzugsweise an der Scheibe (38, 54, 56, 76) oder direkt an dem Isolationsmantel (16) anliegt, wobei das Druckelement (40, 58, 60, 78) an der Scheibe (38, 54, 56, 76) anliegt oder das Druckelement direkt an dem Isolationsmantel (16) anliegt.

4. Schaftbaugruppe (6) nach einem der Ansprüche 1 bis 3, **gekennzeichnet durch** einen vorzugsweise axialfest mit dem Instrumentenschaft (12) verbundenen, insbesondere auf dem Instrumentenschaft (12) aufgenommenen, kapselartigen Adapter (42, 62), innerhalb welchem der proximale Axialanschlag (36, 50, 52, 74) angeordnet ist.

5. Schaftbaugruppe (6) nach Anspruch 4, **dadurch gekennzeichnet, dass** der Adapter (42, 62) einen distalen Anschlag (44, 64), insbesondere in Form eines sich radial nach innen erstreckenden Abschnitts, zur Begrenzung einer distal gerichteten Axialbewegung des proximalen Axialanschlags (36, 50, 52, 74) aufweist, wobei eine Axialposition des distalen Anschlags (44, 64) in Abhängigkeit eines Wärmeausdehnungsverhaltens des Isolationsmantels (16) und/oder des Instrumentenschafts (12) festgelegt ist.

6. Schaftbaugruppe (6) nach Anspruch 4 oder 5, **dadurch gekennzeichnet, dass** der Adapter (42, 62) eine Aufnahmeschale (46, 68) mit einer Einlegeöffnung zum Einlegen des proximalen Axialanschlags (36, 50, 52, 74) aufweist.

7. Schaftbaugruppe (6) nach Anspruch 6, **dadurch gekennzeichnet, dass** die Einlegeöffnung auf einer proximalen Seite des Adapters (42) ausgebildet ist oder dass die Einlegeöffnung auf einer distalen Seite des Adapters (62) ausgebildet ist.

8. Schaftbaugruppe (6) nach Anspruch 6 oder 7, **dadurch gekennzeichnet, dass** der Adapter (62) eine von einer distalen Seite auf die Aufnahmeschale (68) aufsteckbare, insbesondere aufschraubbare, den distalen Anschlag (64) ausbildende Kappe (70) aufweist oder dass der distale Anschlag (44) integral an einer distalen Seite der Aufnahmeschale (46) ausgebildet ist.

9. Schaftbaugruppe (6) nach einem der Ansprüche 1 bis 8, **gekennzeichnet durch** ein den ersten proximalen Axialanschlag (50) axial vorspannendes erstes Druckelement (58) und ein den zweiten proximalen Axialanschlag (52) axial vorspannendes zweites Druckelement (60).

10. Schaftbaugruppe (6) nach Anspruch 9, **dadurch gekennzeichnet, dass** das erste Druckelement (58) und das zweite Druckelement (60) unterschiedliche Federhärten aufweisen.

11. Schaftbaugruppe (6) nach Anspruch 9 oder 10, **dadurch gekennzeichnet, dass** das erste Druckelement (58) und das zweite Druckelement (60) radial geschachtelt angeordnet sind.

12. Schaftbaugruppe (6) nach einem der Ansprüche 1 bis 11, **gekennzeichnet durch** eine den ersten proximalen Axialanschlag (50) ausbildende erste Scheibe (54) und eine den zweiten proximalen Axialanschlag (52) ausbildende zweite Scheibe (56).

13. Schaftbaugruppe (6) nach Anspruch 12, **dadurch gekennzeichnet, dass** die erste Scheibe (54) und die zweite Scheibe (56) axial hintereinander, insbesondere aneinander anliegend, angeordnet sind.

14. Schaftbaugruppe (6) nach Anspruch 13, **dadurch gekennzeichnet, dass** die distal angeordnete Scheibe (56) einen solchen Innendurchmesser oder eine solche Durchführöffnung aufweist, durch den oder die der Isolationsmantel (16) zum axialen Anliegen an der proximal angeordneten Scheibe (54) axial hindurchführbar ist.

15. Chirurgisches Instrument (2), insbesondere elektrochirurgisches Instrument (2) der minimalinvasiven Schaftbauart, mit einer Schaftbaugruppe (6) nach einem der Ansprüche 1 bis 14.

## Claims

1. A surgical shaft assembly (6) of or for a surgical instrument (2), in particular an electrosurgical instrument (2) of the minimally invasive shaft design, at the distal end of which, a tool (4) is coupled or coupleable and at the proximal end of which, a handle (8), in particular in the manner of a pistol-grip handle for actuating the tool (4) is coupleable, with
- an instrument shaft (12) formed in particular from a metal, preferably made of steel;
- an insulation sheath (16) formed of a material different from the instrument shaft (12), in particular made of plastic, preferably made of PEEK, and which is arranged axially displaceably on the instrument shaft (12) and encases the instrument shaft (12) radially on the outside for electrical insulation of the instrument shaft (12), preferably completely on the circumferential side, and
- a proximal axial stop (36, 50, 52, 74) received axially displaceably on the instrument shaft (12) and axially pre-stressed in the distal direction for limiting a proximally directed axial movement of the insulation sheath (16) relative to the instrument shaft (12),
**characterized in that** the shaft assembly (6) comprises two of the proximal axial stops (50, 52), of which a first proximal axial stop (50) serves for limiting the axial movement of the insulation sheath (16) with a first diameter, and a second proximal axial stop (52) serves for limiting the axial movement of the insulation sheath (16) with a second diameter.

2. The surgical shaft assembly (6) according to claim 1, **characterized by** a preferably annular disk (38, 54, 56, 76) forming the proximal axial stop (36, 50, 52, 74).

3. The surgical shaft assembly (6) according to claim 1 or 2, **characterized by** a pressure element (40, 58, 60, 78) axially pre-stressing the proximal axial stop (36, 50, 52, 74), in particular in the form of a spring, preferably a helical spring, which preferably rests against the disk (38, 54, 56, 76) or directly against the insulation sheath (16), wherein
the pressure element (40, 58, 60, 78) rests against the disk (38, 54, 56, 76), or
the pressure element directly rests against the insulation sheath (16).

4. The surgical shaft assembly (6) according to one of claims 1 to 3, **characterized by** a capsule-like adapter (42, 62), preferably axially fixedly connected to the instrument shaft (12), in particular held on the instrument shaft (12), and within which the proximal axial stop (36, 50, 52, 74) is arranged.

5. The surgical shaft assembly (6) according to claim 4, **characterized in that** the adapter (42, 62) has a distal stop (44, 64), in particular in the form of a portion extending radially inward, for limiting a distally directed axial movement of the proximal axial stop (36, 50, 52, 74), wherein
an axial position of the distal stop (44, 64) is fixed as a function of a thermal expansion behavior of the insulation sheath (16) and/or of the instrument shaft (12).

6. The surgical shaft assembly (6) according to claim 4 or 5, **characterized in that** the adapter (42, 62) has a holding tray (46, 68) with an insertion opening for inserting the proximal axial stop (36, 50, 52, 74).

7. The surgical shaft assembly (6) according to claim 6, **characterized in that** the insertion opening is formed on a proximal side of the adapter (42), or **in that** the insertion opening is formed on a distal side of the adapter (62).

8. The surgical shaft assembly (6) according to claim 6 or 7, **characterized in that** the adapter (62) comprises a cap (70) which forms the distal stop (64) and which can be plugged, in particular screwed, onto the holding tray (68) from a distal side, or **in that** the distal stop (44) is integrally formed on a distal side of the holding tray (46).

9. The surgical shaft assembly (6) according to one of claims 1 to 8, **characterized by** a first pressure element (58) axially pre-stressing the first proximal axial stop (50), and a second pressure element (60) axially pre-stressing the second proximal axial stop (52).

10. The surgical shaft assembly (6) according to claim 9, **characterized in that** the first pressure element (58) and the second pressure element (60) have different spring rates.

11. The surgical shaft assembly (6) according to claim 9 or 10, **characterized in that** the first pressure element (58) and the second pressure element (60) are arranged in a radially nested manner.

12. The surgical shaft assembly (6) according to one of claims 1 to 11, **characterized by** a first disk (54) forming the first proximal axial stop (50) and a second disk (56) forming the second proximal axial stop (52).

13. The surgical shaft assembly (6) according to claim 12, **characterized in that** the first disk (54) and the second disk (56) are arranged axially one behind the other, in particular adjacent to each other.

14. The surgical shaft assembly (6) according to claim 13, **characterized in that** the distally arranged disk (56) has such an inner diameter or such a feed-through opening through which the insulation sheath (16) can be axially passed through for axial abutment against the proximally arranged disk (54).

15. A surgical instrument (2), in particular an electrosurgical instrument (2) of the minimally invasive shaft design, comprising a surgical shaft assembly (6) according to one of claims 1 to 14.

## Revendications

1. Ensemble de tige chirurgicale (6) d'un, ou pour un, instrument chirurgical (2), en particulier un instrument électrochirurgical (2) de type à tige mini-invasive, à l'extrémité distale duquel un outil (4) est couplé ou peut être couplé et à l'extrémité proximale duquel une poignée (8), en particulier de type poignée pistolet, peut être couplée pour actionner l'outil (4), avec
- une tige d'instrument (12) conçue en particulier en un métal, de préférence en acier ;
- une gaine isolante (16) conçue dans un matériau différent de celui de la tige d'instrument (12), en particulier en matière plastique, de préférence en PEEK, qui est disposée de manière axialement mobile sur la tige d'instrument (12) et qui enveloppe la tige d'instrument (12) radialement à l'extérieur pour l'isolation électrique de la tige d'instrument (12), de préférence sur tout le côté circonférentiel, et
- une butée axiale proximale (36, 50, 52, 74) logée de manière axialement mobile sur la tige d'instrument (12) et précontrainte axialement dans une direction distale pour limiter un mouvement axial de la gaine isolante (16) dirigé proximalement par rapport à la tige d'instrument (12),
**caractérisé en ce que** l'ensemble de tige (6) présente deux butées axiales proximales (50, 52), dont une première butée axiale proximale (50) sert à limiter le mouvement axial de la gaine isolante (16) avec un premier diamètre et une seconde butée axiale proximale (52) sert à limiter le mouvement axial de la gaine isolante (16) avec un second diamètre.

2. Ensemble de tige (6) selon la revendication 1, **caractérisé par** un disque (38, 54, 56, 76) de préférence annulaire formant la butée axiale proximale (36, 50, 52, 74).

3. Ensemble de tige (6) selon la revendication 1 ou 2, **caractérisé par** un élément de pression (40, 58, 60, 78) précontraignant axialement la butée axiale proximale (36, 50, 52, 74), en particulier sous forme d'un ressort, de préférence un ressort hélicoïdal, lequel s'appuie de préférence contre le disque (38, 54, 56, 76) ou directement contre la gaine isolante (16), dans lequel l'élément de pression (40, 58, 60, 78) repose contre le disque (38, 54, 56, 76) ou l'élément de pression repose directement contre la gaine isolante (16).

4. Ensemble de tige (6) selon l'une quelconque des revendications 1 à 3, **caractérisé par** un adaptateur (42, 62) de type capsule, connecté de préférence de manière axialement fixe à la tige d'instrument (12), en particulier logé sur la tige d'instrument (12), à l'intérieur duquel la butée axiale proximale (36, 50, 52, 74) est disposée.

5. Ensemble de tige (6) selon la revendication 4, **caractérisé en ce que** l'adaptateur (42, 62) présente une butée distale (44, 64), en particulier sous forme d'une section s'étendant radialement vers l'intérieur, pour limiter un mouvement axial dirigé vers l'extrémité distale de la butée axiale proximale (36, 50, 52, 74), dans lequel une position axiale de la butée distale (44, 64) est établie en fonction du présentement de dilatation thermique de la gaine isolante (16) et/ou de la tige d'instrument (12).

6. Ensemble de tige (6) selon la revendication 4 ou 5, **caractérisé en ce que** l'adaptateur (42, 62) présente une coque de logement (46, 68) avec une ouverture d'insertion pour insérer la butée axiale proximale (36, 50, 52, 74).

7. Ensemble de tige (6) selon la revendication 6, **caractérisé en ce que** l'ouverture d'insertion est formée sur un côté proximal de l'adaptateur (42) ou **en ce que** l'ouverture d'insertion est formée sur un côté distal de l'adaptateur (62).

8. Ensemble de tige (6) selon la revendication 6 ou 7, **caractérisé en ce que** l'adaptateur (62) présente un capuchon (70) pouvant être enfiché, en particulier vissé, sur la coque de logement (68) depuis un côté distal et formant la butée distale (64), ou **en ce que** la butée distale (44) est formée d'un seul tenant sur un côté distal de la coque de logement (46).

9. Ensemble de tige (6) selon l'une quelconque des revendications 1 à 8, **caractérisé par** un premier élément de pression (58) précontraignant axialement la première butée axiale proximale (50) et un second élément de pression (60) précontraignant axialement la seconde butée axiale proximale (52).

10. Ensemble de tige (6) selon la revendication 9, **caractérisé en ce que** le premier élément de pression (58) et le second élément de pression (60) présentent des duretés élastiques différentes.

11. Ensemble de tige (6) selon la revendication 9 ou 10, **caractérisé en ce que** le premier élément de pression (58) et le second élément de pression (60) sont disposés de manière emboîtée radialement.

12. Ensemble de tige (6) selon l'une quelconque des revendications 1 à 11, **caractérisé par** un premier disque (54) formant la première butée axiale proximale (50) et un second disque (56) formant la seconde butée axiale proximale (52).

13. Ensemble de tige (6) selon la revendication 12, **caractérisé en ce que** le premier disque (54) et le second disque (56) sont disposés axialement l'un derrière l'autre, en particulier en butée l'un contre l'autre.

14. Ensemble de tige (6) selon la revendication 13, **caractérisé en ce que** le disque distal (56) présente un diamètre intérieur ou une ouverture de passage tels que la gaine isolante (16) peut être introduite axialement à travers celle-ci pour venir en appui axial contre le disque proximal (54).

15. Instrument chirurgical (2), en particulier instrument électrochirurgical (2) de type à tige mini-invasive, avec un ensemble de tige (6) selon l'une quelconque des revendications 1 à 14.
